# EUROPÄISCHE PATENTANMELDUNG

(11) **EP 3 922 243 A2**
(43) Veröffentlichungstag der Anmeldung: **15.12.2021**
(21) Anmeldenummer: 21200711.6
(22) Anmeldetag: 04.10.2021
(51) Int. Cl.: A61K 9/50, A61K 33/06, A61K 9/00

(54) **DIREKTGRANULAT MIT KOMBINATION VON RETARDIERTEM UND SCHNELLFREISETZENDEM MAGNESIUM**

(71) Anmelder: HERMES PHARMA GmbH, 82049 Pullach (DE)
(72) Erfinder: Weiss, Gerd, 81541 München (DE); Haala, Josef, 82049 Pullach (DE); Schiemenz, Wolfgang, 82049 Pullach (DE)
(74) Vertreter: Pharma Patents International AG

(57) **Zusammenfassung**

Erfindungsgemäß wird ein orales Direktgranulat, welches eine kontrollierte Magnesium-Freisetzung mit sowohl retardierten als auch nicht-retardierten Anteilen ermöglicht.

Das Granulat enthält unter anderem Retardpartikel mit einem magnesiumhaltigen Kern und einem lipophilen Überzug, wobei mindestens die Kerne dieser Retardpartikel eine vorgegebene Abriebfestigkeit aufweisen, welche mittels Vibrationssiebung über einen Zeitraum von 60 Minuten bestimmt wird und welche eine Schmelzbeschichtung dieser Kerne ermöglicht. Die Erfindung stellt ferner ein Verfahren zur Herstellung der Retardpartikel mittels Schmelzbeschichtung (Hot-Melt-Coating) bereit.

## Beschreibung

### HINTERGRUND DER ERFINDUNG

Die vorliegende Erfindung betrifft neue Zusammensetzungen, insbesondere orale Direktgranulate, welche den Wirkstoff Magnesium enthalten, insbesondere eine Kombination aus einem Magnesium in retardierter Form sowie in einer schnellerfreisetzenden, nicht-retardierten Form. Die Zusammensetzungen werden unter anderem als Nahrungsergänzungsmittel und/oder als Arzneimittel in der Therapie oder Prävention von Magnesium-Mangelzuständen eingesetzt.

Um auch in Phasen erhöhten Bedarfs eine ausreichende Magnesium-Versorgung des Körpers zu sichern (bspw. bei Stress, Schwangerschaft, Mangelernährung, ausgeprägter sportlicher Betätigung, Diuretika-Therapie oder starkem Alkoholkonsum), empfiehlt es sich mitunter, dieses wichtige Mineral durch externe Quellen zu substituieren. Wie bei vielen wasserlöslichen Wirkstoffen besteht hierbei die Gefahr, dass der Körper überschüssiges Magnesium rasch mit dem Urin wieder ausscheidet, also jene Mengen, die er akut nicht verwenden oder in endogene Speicher einlagern kann. Zudem reagieren einige Personen mit Magenbeschwerden und/oder Durchfall auf höhere freie Magnesium-Konzentrationen im Magen-Darm-Trakt. Eine Formulierung mit zumindest in Teilen kontrollierter Freisetzung der enthaltenen Magnesiumgesamtdosis kann hierbei Abhilfe schaffen und bietet zudem weitere Vorteile wie u.a. eine geringere Einnahmefrequenz.

Für eine anhaltende, möglichst konstante Magnesium-Versorgung des Körpers wurden bereits Formulierungen mit kontrollierter Freisetzung entwickelt; beispielsweise retardierte Freisetzung (verzögerte Freisetzung) oder pulsatile Freisetzung (d.h. in mehreren "Pulsen" über einen längeren Zeitraum). So gibt es beispielsweise im Handel eine 3-lagige Magnesium-Mehrschichttablette mit B-Vitamin-Zusatz (Biolectra^{®} Magnesium 400 mg ultra 3-Phasen-Depot; Hermes Arzneimittel GmbH) welche als Nahrungsergänzungsmittel zur einmal täglichen Einnahme gedacht ist. Die drei Schichten enthalten (i) 200 mg Magnesium (+ Vit. B2 und B12) zur sofortigen Freisetzung,
(ii) 90 mg Magnesium (+ Vit. B1) zur intermediären Freisetzung, sowie
(iii) 110 mg Magnesium (+ Vit. B6) zur Langzeit-Freisetzung. Als Magnesiumverbindungen werden Magnesiumoxid, Magnesiumcarbonat sowie Magnesiumcitrat eingesetzt. Als Hilfsstoffe für die retardierte Freisetzung werden quellbare Polymere wie Hydroxypropylmethylcellulose (HPMC), Hydroxypropylcellulose (HPC) und Polyvinylpolypyrrolidon (PVP) eingesetzt. Leider sind diese Tabletten relativ groß aufgrund der hohen benötigten Mengen der Magnesium-Verbindungen sowie der Hilfsstoffe für deren retardierte Freisetzung (ca. 2 g Tablettengewicht), und dadurch für viele Anwender nicht problemlos oder nur unangenehm schluckbar.

Eine mögliche Abhilfe, bzw. Alternative, für große, schlecht schluckbare Tabletten bieten Brausetabletten oder Pulver zum Auflösen in Wasser oder auch sogenannte orale Direktgranulate. Granulate im engeren Sinne sind sogenannte Haufwerke, die mittels Aggregation oder Agglomeration von Pudern und/oder Pulvern hergestellt werden und somit meist grobkörniger als Puder und Pulver sind (wobei die Grenzen der Partikelgrößen insbesondere von Pulvern und Granulaten überlappen können). Gängige Verfahren zur Granulierung bzw. Aggregation sind dem Fachmann bekannt und in der Literatur beschrieben. Der Begriff "orale Direktgranulate" hingegen wird vom Fachmann breiter verstanden; es ist hierbei meist nicht von Belang, ob das Haufwerk tatsächlich durch eine Aggregation von Pulverpartikeln gewonnen wurde, oder ob einzelne oder mehrere Bestandteile des Direktgranulates anderweitig verarbeitet wurden. Es handelt sich bei oralen Direktgranulaten primär um oral verabreichbare, fließfähige Haufwerke, bevorzugt mit einer Teilchengröße im Bereich von 50 µm bis 500 µm, welche direkt, also in ihrer granulären bzw. partikulären, Form angewendet bzw. verabreicht werden; d.h. ohne hierfür Wasser oder andere trinkbare Flüssigkeiten zum Nachspülen zu benötigen. Sie können bspw. in den Mund und/oder auf die Zunge gestreut und dann leicht geschluckt werden.

Für solche oralen Direktgranulate ist allerdings das Mundgefühl von großer Bedeutung, welches unter anderem mitbestimmt wird durch strukturelle Eigenschaften (bspw. Texturen wie fettig, trocken, hart, formbar, klebrig, krümelig, etc.), durch geometrische Eigenschaften (groß/klein, rund/kantig, etc.) sowie durch Eigenschaften, welche mit dem im Mund-Rachenraum wahrgenommenen Wassergehalt (saftig, trocken, spröde, o.ä.), Temperaturempfindungen (wärmend, kühlend) oder der Reizung freier Nervenenden (scharf, brennend, prickelnd) zusammenhängen. Hier haben sich lipid-basierte Schmelzüberzüge bewährt, da sie häufig ein geringeres Fremdkörpergefühl auf der Zunge hinterlassen.

So beschreibt bspw. EP3042648B1 ein orales Direktgranulat mit Ascorbinsäure-Retardpartikeln aus kristalliner Ascorbinsäure und einem lipid-basierten Überzug, welcher in der Wirbelschicht als Schmelze auf die kristalline Ascorbinsäure aufgetragen wird.

Die Entwicklung eines industriell herstellbaren oralen Direktgranulats, welches eine kontrollierte Magnesium-Freisetzung mit sowohl retardierten als auch nicht-retardierten Anteilen ermöglicht, wurde bisher allerdings unter anderem dadurch erschwert, dass eine Mehrzahl der kommerziell erhältlichen Rohware von Magnesium-Formulierungen - anders als bspw. kristalline Ascorbinsäure - nicht hinreichend physikalisch stabil ist, um dem Schmelzüberzugsprozess (auch Hotmelt-Coating genannt) standzuhalten. Sehr häufig wurde hierbei beobachtet, dass zum Beispiel vorgranulierte Ware unter dem prozess-inhärenten Wärmeinfluss zerfiel und dann die Filter des Wirbelschichtgeräts verstopfte, weit bevor der Schmelzüberzugsprozess ausreichend weit vorangeschritten war. Die Notwendigkeit sehr häufiger Filterwechsel, bzw. Filterreinigungsschritte machten diese Prozesse somit für den Industriemaßstab (bspw. ≥50 kg pro Charge) ungeeignet.

Zudem sind gerade solche Magnesium-Verbindungen, welche sich für den nicht-retardierten Dosisanteil aufgrund ihrer höheren Löslichkeit und Bioverfügbarkeit eher anbieten (z. Bsp. Magnesiumcitrate oder ähnliche Magnesiumsalze organischer Säuren), mehrheitlich sehr sauer oder geschmacklich wenig akzeptabel, und sind somit in einem oralen Direktgranulat schwieriger zu verabreichen, da dieses unverdünnt mit der Zunge in Kontakt kommt.

Der vorliegenden Erfindung liegt somit die Aufgabe zugrunde, eine Zusammensetzung für Magnesiumverbindungen zur Verfügung zu stellen, welche industriell mit wenigen Schritten, wirtschaftlich hergestellt werden kann und eine kontrollierte Freisetzung von Magnesium mit sowohl retardierten als auch nicht-retardierten Anteilen ermöglicht. Die Zusammensetzung sollte leichter und angenehmer verabreichbar sein als gängige Tabletten oder Kapseln, bevorzugt auch ohne zusätzliche Flüssigkeitsgabe. Sie sollte zudem die Stabilität der Magnesiumverbindungen gewährleisten.

Diese Aufgabe wird erreicht durch die erfindungsgemäße feste pharmazeutische oder nutrazeutische Zusammensetzung in Form eines oralen Direktgranulats wie nachfolgend beschrieben.

### ZUSAMMENFASSUNG DER ERFINDUNG

In einem ersten Aspekt betrifft die vorliegende Erfindung eine feste pharmazeutische oder nutrazeutische Zusammensetzung in Form eines oralen Direktgranulats mit mindestens dualer Wirkstofffreisetzung, enthaltend folgende Komponenten:
(a) überzogene Retardpartikel mit einem Magnesium-haltigen Kern und einem LipidÜberzug, wobei der Kern eine Magnesiumverbindung enthält oder aus dieser besteht, und wobei die Retardpartikel das enthaltene Magnesium in retardierter Weise freisetzen;
(b) nicht-retardierte Magnesium-haltige Partikel, wobei die Partikel eine Magnesiumverbindung enthalten oder aus dieser bestehen, und wobei die Partikel das enthaltene Magnesium in nicht-retardierter Weise freisetzen;
(c) einen oder mehrere wasserlösliche Hilfsstoffe ausgewählt aus der Gruppe der Zucker, Zuckeralkohole und Oligosaccharide; und
(d) optional einen oder mehrere weitere Hilfsstoffe;
   dadurch gekennzeichnet, dass mindestens die Magnesium-haltigen Kerne der Retardpartikel der Komponente (a) eine solche Abriebfestigkeit aufweisen, dass nach einer Vibrationssiebung über einen Zeitraum von 60 Minuten:
   - der Feinanteil an Partikeln mit einem Siebdurchmesser von < 200 µm nach einer Siebzeit von 60 Minuten, t₆₀ₘᵢₙ, um maximal 100 Gew.-% höher ist gegenüber dem initialen Feinanteil dieses Siebdurchmessers nach einer Siebzeit von 5 Minuten, t₅ₘᵢₙ; und/oder
   - der Anteil an Partikeln mit einem Siebdurchmesser von 200 µm ≤ x < 800 µm nach einer Siebzeit von 60 Minuten, t₆₀ₘᵢₙ, um maximal 18 Gew.-% niedriger ist gegenüber dem initialen Anteil dieses Siebdurchmessers nach einer Siebzeit von 5 Minuten, t₅ₘᵢₙ.

In einem zweiten Aspekt betrifft die vorliegende Erfindung ein Verfahren zur Herstellung einer festen pharmazeutischen oder nutrazeutischen Zusammensetzung gemäß dem ersten Aspekt der Erfindung, wobei das Verfahren die folgenden Schritte enthält bzw. umfasst:
(i) Bereitstellen eines Magnesium-haltigen Kerns, wobei der Kern eine Magnesiumverbindung enthält oder aus dieser besteht;
(ii) Bereitstellen eines geschmolzenen Überzugsmaterials enthaltend ein Lipid;
(iii) Fluidisieren der Magnesium-haltigen Kerne;
(iv) Besprühen der fluidisierten Magnesium-haltigen Kerne mit dem geschmolzenen Überzugsmaterial;
(v) Abkühlen der überzogenen Magnesium-haltigen Kerne, so dass das Lipid erstarrt und Retardpartikel gemäß Komponente (a) erhalten werden, welche das enthaltene Magnesium in retardierter Weise freisetzen; und
(vi) Mischen der so als Komponente (a) erhaltenen Retardpartikel mit den folgenden weiteren Komponenten:
   (b) nicht-retardierte Partikel mit einem zweiten Magnesium-haltigen Kern, wobei der Partikel eine Magnesiumverbindung enthält oder aus dieser besteht, und wobei die Partikel das enthaltene Magnesium in nicht-retardierter Weise freisetzen;
   (c) einen oder mehrere wasserlösliche Hilfsstoffe ausgewählt aus der Gruppe der Zucker, Zuckeralkohole und Oligosaccharide; und
   (d) optional weiteren Hilfsstoffen;
      dadurch gekennzeichnet, dass mindestens die Magnesium-haltigen Kerne der Retardpartikel der Komponente (a) eine solche Abriebfestigkeit aufweisen, dass nach einer Vibrationssiebung über einen Zeitraum von 60 Minuten:
      - der Feinanteil an Partikeln mit einem Siebdurchmesser von < 200 µm nach einer Siebzeit von 60 Minuten, t₆₀ₘᵢₙ, um maximal 100 Gew.-% höher ist gegenüber dem initialen Feinanteil dieses Siebdurchmessers nach einer Siebzeit von 5 Minuten, t₅ₘᵢₙ; und/oder
      - der Anteil an Partikeln mit einem Siebdurchmesser von 200 µm ≤ x < 800 µm nach einer Siebzeit von 60 Minuten, t₆₀ₘᵢₙ, um maximal 18 Gew.-% niedriger ist gegenüber dem initialen Anteil dieses Siebdurchmessers nach einer Siebzeit von 5 Minuten, t₅ₘᵢₙ.

In einem weiteren Aspekt der Erfindung betrifft die vorliegende Erfindung eine feste pharmazeutische oder nutrazeutische Zusammensetzung in Form eines oralen Direktgranulats mit mindestens dualer Wirkstofffreisetzung, welche(s) mittels des Verfahrens gemäß dem zweiten Aspekt der Erfindung hergestellt wird.

### KURZBESCHREIBUNG DER ABBILDUNGEN

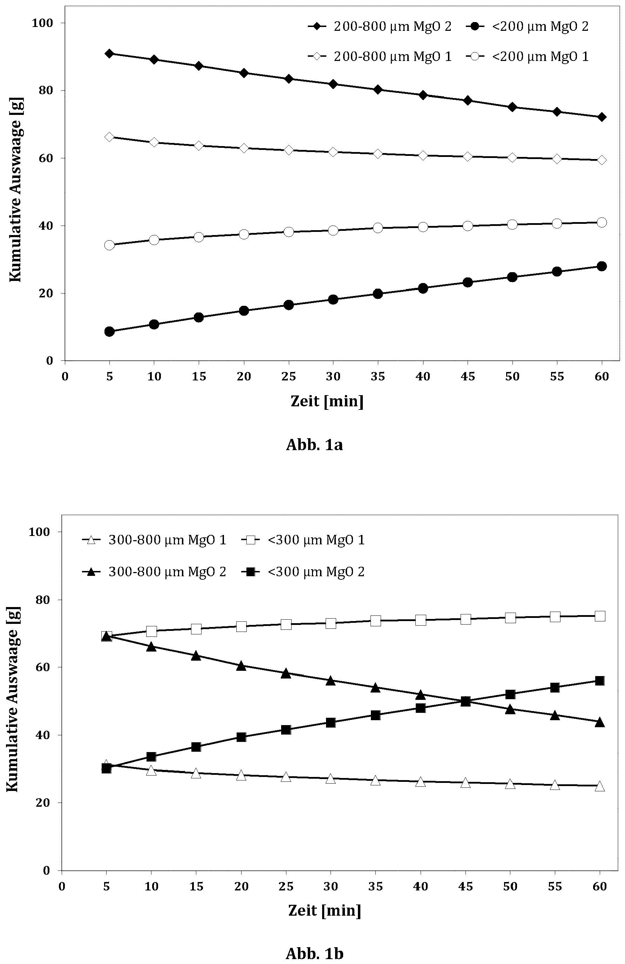

**Abb. 1a** und **Abb. 1b** zeigen die Ergebnisse der Vibrationssiebung über eine Zeit von 60 Minuten für zwei der getesteten Rohwaren von granuliertem Magnesiumoxid (MgO) Kernen (hier ,Heavy Magnesium Oxide EP' von Kyowa Chemical Industry Co., Ltd Japan, nachstehend als ,MgO 1' bezeichnet; sowie MagGran^{®} MO der Magnesia GmbH Deutschland; nachstehend ,MgO 2').

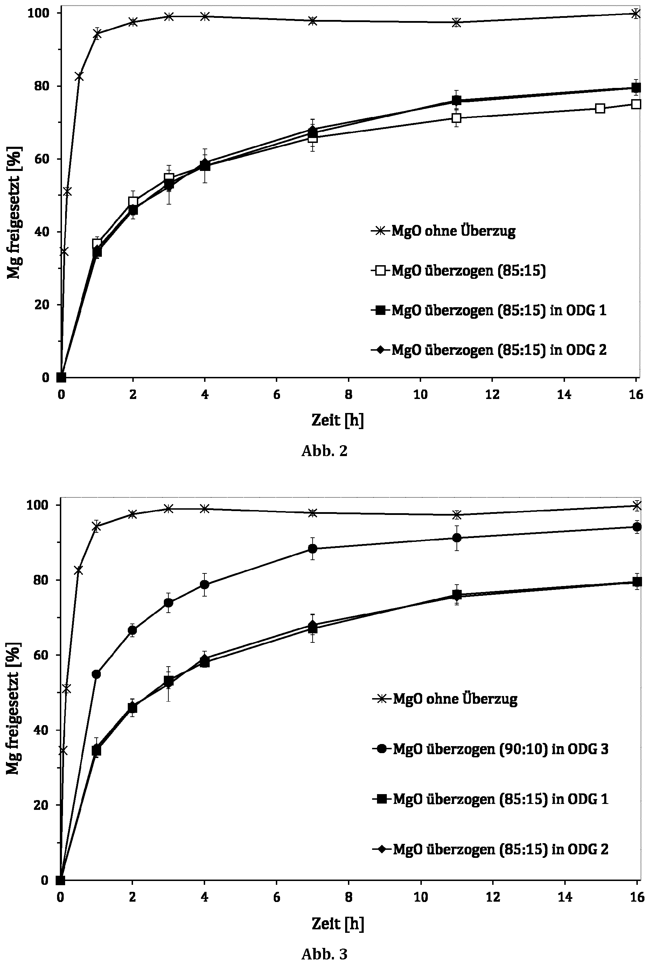

**Abb. 2** zeigt den Einfluss der Formulierung auf die Magnesium-Freisetzung in 0.1 N Salzsäure bei 37 °C für nicht überzogene MgO-Kerne, überzogene MgO-Kerne (Überzugslevel 15 Gew.-%) und erfindungsgemäße orale Direktgranulate (ODG), welche unter anderem diese überzogenen MgO-Kerne enthalten.

**Abb. 3** zeigt den Einfluss des Überzugslevels auf die Magnesium-Freisetzung in 0.1 N Salzsäure bei 37 °C für nicht überzogene MgO-Kerne, und erfindungsgemäße orale Direktgranulate (ODG), welche unter anderem überzogene MgO-Kerne mit einem Überzugslevel von 10 Gew.-% (ODG 3) und 15 Gew.-% (ODG 1 und ODG 2) enthalten.

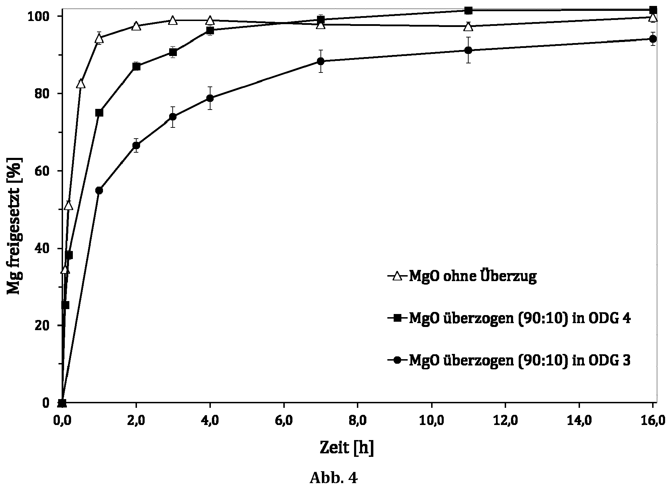

**Abb. 4** zeigt den Einfluss der Formulierung auf die Magnesium-Freisetzung in 0.1 N Salzsäure bei 37 °C für nicht überzogene MgO-Kerne, und erfindungsgemäße orale Direktgranulate (ODG), welche unter anderem überzogene MgO-Kerne mit einem Überzugslevel von 10 Gew.-% enthalten sowie verschiedene Anteile nicht-retardiertes Magnesium, wobei ODG 3 weniger nicht-retardiertes Magnesium enthält als ODG 4.

### DETAILLIERTE BESCHREIBUNG DER ERFINDUNG

In einem ersten Aspekt betrifft die vorliegende Erfindung eine feste pharmazeutische oder nutrazeutische Zusammensetzung in Form eines oralen Direktgranulats mit mindestens dualer Wirkstofffreisetzung, enthaltend folgende Komponenten:
(a) überzogene Retardpartikel mit einem Magnesium-haltigen Kern und einem LipidÜberzug, wobei der Kern eine Magnesiumverbindung enthält oder aus dieser besteht, und wobei die Retardpartikel das enthaltene Magnesium in retardierter Weise freisetzen;
(b) nicht-retardierte Magnesium-haltige Partikel, wobei die Partikel eine Magnesiumverbindung enthalten oder aus dieser bestehen, und wobei die Partikel das enthaltene Magnesium in nicht-retardierter Weise freisetzen;
(c) einen oder mehrere wasserlösliche Hilfsstoffe ausgewählt aus der Gruppe der Zucker, Zuckeralkohole und Oligosaccharide; und
(d) optional einen oder mehrere weitere Hilfsstoffe;
   dadurch gekennzeichnet, dass mindestens die Magnesium-haltigen Kerne der Retardpartikel der Komponente (a) eine solche Abriebfestigkeit aufweisen, dass nach einer Vibrationssiebung über einen Zeitraum von 60 Minuten:
   - der Feinanteil an Partikeln mit einem Siebdurchmesser von < 200 µm nach einer Siebzeit von 60 Minuten, t₆₀ₘᵢₙ, um maximal 100 Gew.-% höher ist gegenüber dem initialen Feinanteil dieses Siebdurchmessers nach einer Siebzeit von 5 Minuten, t₅ₘᵢₙ; und/oder
   - der Anteil an Partikeln mit einem Siebdurchmesser von 200 µm ≤ x < 800 µm nach einer Siebzeit von 60 Minuten, t₆₀ₘᵢₙ, um maximal 18 Gew.-% niedriger ist gegenüber dem initialen Anteil dieses Siebdurchmessers nach einer Siebzeit von 5 Minuten, t₅ₘᵢₙ.

Im Zusammenhang mit der Erfindung bezeichnet der Begriff ,Retardpartikel' solche Partikel, die einen Wirkstoff, hier eine Magnesium-Verbindung, und mindestens einen Hilfsstoff in Form eines Überzugs enthalten, und die so aufgebaut sind, dass der Hilfsstoffüberzug eine langsame Freisetzung des Wirkstoffs bewirkt (sog. Reservoir-Systeme).

In einer spezifischen Ausführungsform weisen mindestens die Magnesium-haltigen Kerne der Retardpartikel der Komponente (a) eine solche Abriebfestigkeit auf, dass nach einer Vibrationssiebung über einen Zeitraum von 60 Minuten der Feinanteil an Partikeln mit einem Siebdurchmesser von < 200 µm nach einer Siebzeit von 60 Minuten, t₆₀ₘᵢₙ, um maximal 50 Gew.-%, bevorzugt um maximal 20 Gew.-%, höher ist gegenüber dem initialen Feinanteil dieses Siebdurchmessers nach einer Siebzeit von 5 Minuten, t₅ₘᵢₙ; und/oder der Anteil an Partikeln mit einem Siebdurchmesser von 200 µm ≤ x < 800 µm nach einer Siebzeit von 60 Minuten, t₆₀ₘᵢₙ, um maximal 15 Gew.-%, bevorzugt um maximal 12 Gew.-%, niedriger ist gegenüber dem initialen Anteil dieses Siebdurchmessers nach einer Siebzeit von 5 Minuten, t₅ₘᵢₙ.

In einer weiteren Ausführungsform weisen mindestens die Magnesium-haltigen Kerne der Retardpartikel der Komponente (a) eine solche Abriebfestigkeit auf, dass nach einer Vibrationssiebung über einen Zeitraum von 60 Minuten der Anteil an Partikeln mit einem Siebdurchmesser von < 300 µm nach einer Siebzeit von 60 Minuten, t₆₀ₘᵢₙ, um maximal 55 Gew.-%, bevorzugt um maximal 35 Gew.-%, weiter bevorzugt um maximal 15 Gew.-%, höher ist gegenüber dem initialen Anteil dieses Siebdurchmessers nach einer Siebzeit von 5 Minuten, t₅ₘᵢₙ; und/oder der Anteil an Partikeln mit einem Siebdurchmesser von 300 µm ≤ x < 800 µm nach einer Siebzeit von 60 Minuten, t₆₀ₘᵢₙ, um maximal 30 Gew.-%, bevorzugt um maximal 27 Gew.-%, weiter bevorzugt um maximal 24 Gew.-%, niedriger ist gegenüber dem initialen Anteil dieses Siebdurchmessers nach einer Siebzeit von 5 Minuten, t₅ₘᵢₙ.

Natürlich können optional auch die nicht-retardierten Magnesium-haltigen Partikel eine solche Abriebfestigkeit wie oben beschrieben aufweisen. Dies wäre unter anderem von Vorteil, wenn bspw. das Auftragen eines wasserlöslichen, nicht-retardierenden Überzugs auf die nicht-retardierten Magnesium-haltigen Partikel oder ein Granulieren derselben in der Wirbelschicht angedacht ist.

Die Vibrationssiebung kann bspw. mit 100 g der Magnesium-haltigen Kerne (gesamte zu siebende Menge) und je 1 g Siebhilfe pro Siebboden sowie bei einer Vibrationsamplitude von 80/min durchgeführt werden. Als Siebhilfe können hierbei bspw. abriebfeste Silicagel-Kugeln verwendet werden, welche einen Durchmesser von 2-5 mm und eine Schüttdichte von ungefähr 750 kg/m³ aufweisen. Die Dauer der erfindungsgemäßen Vibrationssiebung wurde auf 60 min festgelegt (mit mindestens einer Zwischenwägung bei 5 min), da dies ungefähr dem Zeitrahmen eines gängigen Schmelzbeschichtungsverfahrens entspricht; sprich so lange sind die Kerne im Allgemeinen dem mechanischen Stress der Wirbelschicht ausgesetzt und müssen eine hinreichende Abriebfestigkeit aufweisen. In Fällen, die ein deutlich längeres Sprühverfahren erfordern, könnte theoretisch eine längere Siebdauer als 60 Minuten angedacht werden. Allerdings ist dies häufig nicht nötig, da die Magnesium-haltigen Kerne im Allgemeinen nach 60 Minuten bereits so weit überzogen sind, dass der Überzug die Kerne stabilisiert und die Auswirkungen des mechanischen Stresses auf das Kernmaterial immer geringer werden.

Wie oben an erwähnt war in der Vergangenheit die Entwicklung eines industriell herstellbaren oralen Direktgranulats mit schmelzüberzogenen Magnesium-Kernen dadurch erschwert, dass viele, der kommerziell erhältlichen Magnesium-Formulierungen nicht hinreichend stabil schien, um dem Schmelzüberzugsprozess standzuhalten. So zerfiel bspw. vorgranulierte Ware und verstopfte die Filter des Wirbelschichtgeräts, weit bevor der Schmelzüberzugsprozess ausreichend weit vorangeschritten war, was sehr häufige Filterwechsel, bzw. Filterreinigungsschritte, nötig machte, und somit den Prozess ungeeignet für den Industriemaßstab (bspw. ≥50 kg pro Charge). Zudem waren die entstehenden Auflagerungen von verkrustetem Kern-Abrieb und Sprühmaterial auf den Innenwänden des Wirbelschicht-Geräts so ausgeprägt und fest, dass stets sehr zeitaufwändige Reinigungsschritte nötig waren.

Der Einsatz der Vibrationssiebung, und insbesondere der Einsatz der Vibrationssiebung mit der beschriebenen Siebhilfe, ermöglicht es, die mechanischen Belastungen eines Wirbelschicht-Coating-Prozesses zu simulieren, insbesondere die eines Wirbelschicht-Coating-Prozesses bei dem ein Schmelzüberzug (Hotmelt-Coating) aufgebracht wird. Auf diese Weise ist es möglich, vorab die Abriebfestigkeit der für die Herstellung der Retardpartikel vorgesehenen Magnesium-haltigen Kerne zu bestimmen und somit eine Aussage zu treffen, ob diese eine ausreichende Stabilität für den Überzugsprozess aufweisen. Dadurch können unnötige Materialverluste vermieden werden sowie die Leerzeiten (in denen bspw. das Überzugsequipment gereinigt werden muss) reduziert werden, da nur solche Chargen der Magnesium-haltigen Kerne überzogen werden, die die oben beschriebenen Anforderungen an die Abriebfestigkeit aufweisen. Für die Erfindung ist es hierbei unerheblich, ob die Abriebfestigkeit der für die Herstellung der Retardpartikel vorgesehenen Magnesium-haltigen Kerne (ausreichend oder nicht ausreichend) das Resultat von Chargen-Variabilitäten eines einzelnen Anbieters ist, oder durch Chargen verschiedener Anbieter verursacht ist.

In einer Ausführungsform werden die erfindungsgemäßen Magnesium-haltigen Kerne der Retardpartikel mittels Trockenkompaktierung hergestellt.

In einer der bevorzugten Ausführungsform weisen die Magnesium-haltigen Kerne der Retardpartikel eine mediane Teilchengröße (D50), gemessen mittels dynamischer Bildanalyse zwischen 150 µm und 300 µm auf, bevorzugt zwischen 165 µm und 285 µm, weiter bevorzugt zwischen 180 µm und 270 µm.

Alle hierin verwendeten Partikelgrößen-Angaben - sowohl gemessene Werte als auch aus gemessenen Werten berechnete/abgeleitete Werte - beziehen sich auf Werte, die mittels dynamischer Bildanalyse ermittelt wurden; z. Bsp. mit dem Camsizer^{®} XT Gerät der Retsch Technology GmbH, Haan, Deutschland ausgestattet mit der X-Jet Plug-in Kartusche sowie der dazugehörigen Analyse-Software. Der Camsizer^{®}-Aufbau verwendet eine dynamische Bildgebungstechnik, bei der Partikelproben durch Druckluft zerstreut, durch einen von gepulsten LED-Lichtquellen beleuchteten Spalt geleitet und ihre Bilder (genauer ihre Projektionen) hierbei von zwei Digital-Kameras aufgezeichnet und dann in Bezug auf Größe und Form analysiert werden. Auf diese Weise können eine Vielzahl von Längen- und Breitendeskriptoren für die Partikel bestimmt werden, einschließlich der mittleren Partikelgröße, der medianen Partikelgröße (D50), sowie die sog. D10- und D90-Werte. Das Gerät Camsizer^{®} XT wird für die Partikelgrößenbestimmung gemäß der Erfindung bevorzugt, da es die genaue und reproduzierbare Analyse von Partikelgrößen von feinen Pulvern bis hinunter zu 1 µm ermöglicht. Dies sollte jedoch nicht so missverstanden werden, dass die Verwendung von Laserbeugung oder anderen etablierten Methoden zur Partikelgrößenbestimmung im erforderlichen Mikrometerbereich ausgeschlossen wäre; jedoch sind in Fällen, in denen die mit anderen Verfahren ermittelten Partikelgrößen von den mittels Camsizer^{®} ermittelten Werten abweichen, die mit dem Camsizer^{®}- ermittelten Werte maßgebend.

Die oben genannten Partikelgrößen der Magnesium-haltigen Kerne sind insofern vorteilhaft, dass die daraus resultierenden entsprechenden Retardpartikel auch nach Auftrag des zur Retardierung benötigten Überzugs noch eine angenehme Partikelgröße behalten, die bei einer direkten oralen Applikation nicht als grobkörnig oder anderweitig unangenehm empfunden wird. Die Partikelgröße von oralen Direktgranulaten sollte im Allgemeinen 600 µm, bevorzugt 500 µm; nicht überschreiten, um ein Fremdkörpergefühl im Mund zu vermeiden und den Kaureflex gering zu halten.

In einer weiteren bevorzugten Ausführungsform weisen die Magnesium-haltigen Kerne der Retardpartikel eine unimodale Partikelgrößenverteilung auf; sprich eine Partikelgrößenverteilung, die im Verteilungsdiagramm nur einen einzelnen Peak aufweist. Dies deutet im Allgemeinen auf eine überwiegend einheitliche Partikelgröße hin. Weiterhin bevorzugt sind Ausführungsformen, in denen die Magnesium-haltigen Kerne der Retardpartikel eine so enge Partikelgrößenverteilung aufweisen, dass der Quotient (D90-D10) / D50 unter 1,60 liegt, bevorzugt unter 1,50, und weiterhin bevorzugt unter 1,40. Da oberhalb von D90 und unterhalb von D10 eher die Ausreißer der Partikelgrößenverteilung zu finden sind, deutet ein Quotient(D90-D10) / D50, der nahe an eins liegt, daraufhin, dass die Mehrzahl der Partikel eine Größe nahe der medianen Partikelgröße (D50) aufweist.

Es ist weiterhin vorteilhaft, wenn mindestens die Magnesiumverbindung in den Retardpartikeln der Komponente (a) in ihrer wasserfreien Form einen Magnesiumgehalt von 15 Gew.-% oder mehr aufweist, bevorzugt 25 Gew.-% oder mehr, weiterhin bevorzugt 50 Gew.-% oder mehr. Dies ist insofern von Vorteil, dass auf diese Weise die Menge und/oder Größe der "Retard-Kerne" geringgehalten werden kann, und die hieraus hergestellten Retardpartikel auch nach Auftrag Überzugs noch eine angenehme Partikelgröße behalten wie oben beschrieben. Optional kann natürlich auch die Magnesiumverbindung in den nicht-retardierten Partikeln der Komponente (b) in ihrer wasserfreien Form einen Magnesiumgehalt von 15 Gew.-% oder mehr, bevorzugt 25 Gew.-% oder mehr, weiterhin bevorzugt 50 Gew.-% oder mehr aufweisen. In einer Ausführungsform ist die Magnesiumverbindung in den Retardpartikeln ausgewählt aus Magnesiumoxid (MgO), Magnesiumcarbonat (MgCO₃), Magnesiumchlorid (MgCl₂), Magnesiumhydrogenphosphat (MgHPO₄) und/oder Magnesiumacetat (Mg(CH₃COO)₂).

Optional kann der Kern der Retardpartikel einen oder mehrere Hilfsstoffe enthalten, bspw. im Falle einer vorgranulierten Magnesiumhaltigen Rohware eine kleine Menge eines wasserlöslichen Bindemittels, das von Herstellerseite zugesetzt sein kann.

In einer Ausführungsform ist die Magnesiumverbindung im Kern der Retardpartikel Magnesiumoxid (MgO). In einer spezifischen Ausführungsform besteht der Kern in den Retardpartikeln im Wesentlichen aus Magnesiumoxid (MgO).

In einer der bevorzugten Ausführungsform enthält der Überzug der Retardpartikel ein Lipid mit einem Schmelzpunkt von mindestens 50 °C, oder besteht im Wesentlichen hieraus; bevorzugt ein Lipid mit einem Schmelzpunkt von mindestens 60 °C. Im Falle, dass der Überzug mehrere Lipide enthält, hat mindestens eines der Lipide einen Schmelzpunkt von mindestens 50 °C, bevorzugt mindestens 60 °C. Ferner, im nicht unüblichen Fall, dass ein Lipid keinen scharfen Schmelzunkt, sondern einen Schmelzbereich aufweist, ist unter dem Schmelzpunkt im Sinne der Erfindung die Untergrenze des Schmelzbereichs zu verstehen, also die Temperatur, bei der das Lipid während des Aufheizens zu schmelzen beginnt.

Die Erfinder haben festgestellt, dass sich insbesondere Lipide mit einem höheren Schmelzpunkt von mindestens 50 °C, bevorzugt mindestens 60 °C, hervorragend für orale Direktgranulate eignen. Diese Lipide sind zum einen ausreichend fest, um den Magnesium-haltigen Kern der Retardpartikel stabil zu umhüllen und werden bei Körpertemperatur noch nicht (an)geschmolzen. Im Unterschied zu Lipiden mit Schmelzpunkten unter 50 °C oder ähnlichen hydrophoben Umhüllungen aus Wachsen oder Silikonen hinterlassen die erfindungsgemäßen Lipide zudem keinen seifigen oder fettigen (Geschmacks) Eindruck im Mund, was für eine Zusammensetzung in Form eines oralen Direktgranulats wichtig ist, da dieses üblicherweise länger im Mund verbleibt als bspw. eine perorale Tablette und dort nicht durch zusätzliche Flüssigkeiten verdünnt wird.

Gleichzeitig werden die erfindungsgemäßen Lipidüberzüge im Vergleich mit konventionellen Polymer-Überzügen (wie bspw. aus Celluloseethern) im Mund und/oder auf und unter der Zunge subjektiv als weniger hart und weniger "plastikartig" wahrgenommen; erzeugen also weniger Fremdkörpergefühl. All dies trägt maßgeblich zu dem gewünschten angenehmen Mundgefühl des erfinderischen oralen Direktgranulates bei.

Der Lipidüberzug kann optional einen oder mehrere weitere Hilfsstoffe enthalten, mit denen seine Eigenschaften hinsichtlich Verarbeitbarkeit, Geschmack, Aussehen, Stabilität oder Wirkstofffreisetzung angepasst werden. Dies können z. Bsp. wasserlösliche Substanzen sein, Substanzen mit einer geringeren Schmelztemperatur, quellbare Substanzen und/oder Netzmittel wie bspw. Polysorbate (z. Bsp. Tween^{®}). Ein Ziel dieser Hilfsstoffe im Lipidüberzug - sofern eingesetzt - ist es, die Stabilität des Filmes zu unterstützen und ggf. variablere Freisetzungsprofile zu ermöglichen. Beispiele für Hilfsstoffe, welche die Verarbeitbarkeit von Lipiden und Lipidmischungen potenziell verbessern können, sind Emulgatoren, vor allem solche mit mittlerem oder niedrigem HLB-Wert (hydrophilic-lipophilic balance) von etwa 12 oder weniger. Prinzipiell sollten hierbei natürlich nur solche Substanzen gewählt werden, die zum einen den Geschmackseindruck nicht negativ beeinflussen, und zum anderen kein Inkompatibilitätsrisiko mit weiteren Überzugsbestandteilen, im Speziellen mit dem/den Lipid(en), aufweisen (bspw. zu Phasentrennungen führen könnten).

Bevorzugt besteht der Überzug jedoch überwiegend aus einem oder mehreren Lipiden, das heißt zu mindestens 70 Gew.-%. Ebenso bevorzugt sind Überzüge mit mindestens 80 Gew.-% Lipid(en), mit mindestens 90 Gew.-% Lipid(en), oder solche, die praktisch ausschließlich aus Lipid(en) bestehen.

In einer Ausführungsform handelt es sich bei den Lipiden im Überzug der Retardpartikel um Triglyceride (auch Triacylglyceride, Glycerol-Triester oder seltener auch Neutralfette genannt), sprich dreifache Ester von Glycerol mit drei Fettsäuremolekülen.

In einer weiteren Ausführungsform enthält der Überzug der Retardpartikel mindestens 70 Gew.-%, oder mindestens 80 Gew.-%, oder mindestens 90 Gew.-% eines Triglycerids. In einer spezifischen Ausführungsform handelt es sich um Triglyceride natürlicher Fettsäuren, bspw. Palmitinsäure, Stearinsäure oder Arachidon-säure. In einer weiteren spezifischen Ausführungsform handelt es sich bei den Lipiden im Überzug um möglichst reine Formen einfacher Triglyceride, sprich Triglyceride die mindestens 80 Gew.-% und bevorzugt mindestens 90 Gew.-% eines einfachen Triglycerids enthalten, und nur wenig gemischte- und/oder Partialglyceride. Beispiele für solche einfachen Triglyceride sind Glyceroltripalmitat, Glyceroltristearat oder Glyceroltriarachidat. Optional können die Triglyceride auch miteinander gemischt werden.

In einer der bevorzugten Ausführungsformen enthält der Überzug der Retardpartikel Glyceroltripalmitat und/oder Glyceroltristearat, oder besteht im Wesentlichen hieraus; z. Bsp. mit einem Überzug aus Dynasan^{®}116 oder 118. In einer spezifischen Ausführungsform enthält der Überzug der Retardpartikel min. 70 Gew.-%, oder min. 80 Gew.-%, oder min. 90 Gew.-% Glyceroltripalmitat und/oder Glyceroltristearat. In einer der bevorzugten Ausführungsformen, besteht der Überzug der Retardpartikel im Wesentlichen aus Glyceroltripalmitat und/oder Glyceroltristearat. Einer der Vorzüge von Überzügen, welche zu mindestens 90 Gew.-% aus Glyceroltripalmitat und/oder Glyceroltristearat wie bspw. Dynasan^{®}116 oder 118 bestehen, ist, dass sie synthetisch gewonnen werden und häufig reiner sind als die Mehrzahl natürlicher oder partial-synthetischer Lipide; also z. Bsp. weniger gemischte Triglyceride oder Partialglyceride aufweisen, und/oder klarer definierte Schmelzpunkte haben. Dies wirkt sich vorteilhaft auf ihre Verarbeitung aus, da bspw. eine zu sprühende Schmelze nicht unnötig hoch erhitzt werden muss, um alle Bestandteile des Lipids vollständig aufzuschmelzen.

Im Gegensatz hierzu muss bei Lipiden mit breiteren Schmelzbereichen üblicherweise noch oberhalb des oberen Wertes dieses Schmelzbereichs gearbeitet werden, um eine homogene Zusammensetzung der Schmelze zu gewährleisten und ein mögliches Verstopfen der Sprühdüse(n) zu verhindern. Solche ,übererhitzten' Schmelzen härten auf den zu besprühenden Magnesium-haltigen Kernen dann langsamer aus, formen also den gewünschten Überzug weniger zügig. Zudem kann die Agglomerationstendenz durch das langsamere Aushärten erhöht sein. Weiterhin sind Entmischungen der Schmelze während des Erstarrens auf den zu besprühenden Magnesium-haltigen Kernen möglich, wenn die höherschmelzenden Komponenten der Schmelze zuerst aushärten.

Darüber hinaus liegen die Überzüge mit mindestens 90 Gew.-% Glyceroltripalmitat und/oder Glyceroltristearat nach der Verarbeitung bereits in ihrer stabileren β-Modifikation vor, so dass sich die Freisetzung während der Lagerung üblicherweise nicht, bzw. nur marginal ändert. Die so erhaltenen Retardpartikel sind also deutlich lager-stabiler.

In einer weiteren bevorzugten Ausführungsform besteht der Überzug der Retardpartikel im Wesentlichen aus Glyceroltristearat. Insbesondere Glyceroltristearat (Schmelzpunkt Mₚ ca. 70-73 °C) hat sich in Versuchen aufgrund seines höheren Schmelzpunktes als besonders angenehm im Mund herausgestellt und hinterlässt überraschenderweise deutlich weniger unangenehm seifige und/oder fettige (Geschmacks) Eindrücke als andere Triglyceride mit einem Schmelzpunkt deutlich oberhalb der Körpertemperatur wie z. Bsp. Trilaurin (Mₚ ca. 44-47 °C). Zudem ist Glyceroltristearat deutlich geschmacksneutraler als eine Vielzahl natürlicher oder partial-synthetischer Lipide. Es hinterlässt bspw. anders als gehärteter Rindertalg (Mₚ ca. 60 °C) oder gehärtetes Rizinusöl (Mₚ 79-80 °C) keinen ,deftigen' oder respektive ,kratzenden' Geschmackseindruck auf der Zunge. Auch viele neutrale gehärtete Öle (wie Raps oder Soja) können unter Umständen in Direktgranulaten als geschmacklich unangenehm bzw. unpassend wahrgenommen werden und harmonieren nicht mit frischen und/oder fruchtigen Aromen wie Orange, Zitrone, Kirsche, Beere, Tropenfrüchte oder dergleichen.

Die Freisetzung lässt sich neben der Zusammensetzung des Überzugs, auch über dessen Stärke (oder Dicke) steuern. Die Stärke ergibt sich wiederum aus der Auftragsmenge des Überzugsmaterials und aus der Oberfläche der zu überziehenden Partikel. Je nach der Größe der Magnesium-haltigen Kerne und der sich daraus ergebenden Oberfläche ist demnach die Auftragsmenge des Überzugsmaterials zu wählen, mit dem die gewünschte Retardierung erzielt wird. In einer der bevorzugten Ausführungsformen liegt das Gewichtsverhältnis zwischen Kern und Überzug in den Retardpartikeln zwischen 60:40 und 95:5, oder zwischen 70:30 und 90:10; bspw. 75:25, 80:20, 85:15 oder 90:10. Das konkrete Gewichtsverhältnis zwischen Kern und Überzug kann nach Maßgabe der angestrebten Freisetzungskinetik bzw. Retardierung und der Zusammensetzung angepasst werden. Hierbei ist zu beachten, dass sich die benötigte Menge an Überzugsmaterial selbst für ein und denselben Lipidüberzug leicht ändern kann, wenn bspw. die Partikelform und -größe und/oder die Porosität des Überzugsgutes variieren.

In einer der bevorzugten Ausführungsformen sind die Retardpartikel mit einem Überzug versehen, der zu mindestens 90 Gew.-% aus Glyceroltripalmitat und/oder Glyceroltristearat besteht, und bei dem das Gewichtsverhältnis zwischen Kern und Überzug zwischen 60:40 und 95:5 liegt. In einer ebenfalls bevorzugten Ausführungsform sind die Retardpartikel mit einem Überzug versehen, der zu mindestens 90 Gew.-% aus Glyceroltristearat besteht, oder der optional im Wesentlichen hieraus besteht, und bei dem das Gewichtsverhältnis zwischen Kern und Überzug zwischen 70:30 und 90:10 liegt, bevorzugt zwischen 80:20 und 90:10.

Im Zusammenhang mit der oben erwähnten zu überziehenden Oberfläche ist zudem zu beachten, dass Magnesium-haltige Kerne mit unzureichender Abriebfestigkeit, wie bspw. von den Erfindern in früheren Schmelzbeschichtungsversuchen verwendet, nicht allein aufgrund der sehr arbeits- und zeitintensiven Arbeitsschritte kritisch sind (verstopfte Filtern, häufige und schwierige Reinigung), sondern vor allem ein Problem darstellen, da sich durch die fortschreitende Zerkleinerung eines unzureichend stabilen Kernmaterials in der Wirbelschicht die zu überziehende Oberfläche in kaum kalkulierbarer Weise verändert und somit ein reproduzierbares Schmelzbeschichtungsverfahren nahezu unmöglich wird.

*In vitro* lässt sich das Freisetzungsprofil gemäß den gängigen Arzneibüchern mit einer genormten Freisetzungsapparatur unter standardisierten Bedingungen bestimmen. Erfindungsgemäß wird das Freisetzungsprofil bevorzugt in einer Freisetzungs-Apparatur mit Blattrührer (USP Dissolution Apparatus 2) bspw. in 900 mL 0.1 N Salzsäure (HCl) bei 37 °C und einer Rührgeschwindigkeit von 75 UPM bestimmt. Unter diesen Bedingungen zeigt die erfindungsgemäße Zusammensetzung eine Retardierung über mindestens ca. 4-6 Stunden, vorzugsweise über mindestens ca. 6-8 Stunden; d.h. vor Ablauf des jeweiligen Zeitraums ist der Wirkstoff noch nicht vollständig freigesetzt.

In einer der bevorzugtem Ausführungsformen ist die Stärke des Überzugs in den Retardpartikeln so gewählt, dass die Retardpartikel innerhalb von 4 Stunden, bevorzugt innerhalb von 5 Stunden, weiter bevorzugt innerhalb von 6 Stunden, nicht mehr als 85 % des in ihnen enthaltenen Magnesiums freisetzen, gemessen in einer Freisetzungs-Apparatur mit Blattrührer (USP Dissolution Apparatus 2) in 900 mL 0.1 N Salzsäure bei 37 °C und einer Rührgeschwindigkeit von 75 UPM. Längere Retardierungen durch die Retardpartikel sind häufig nicht mehr zielführend, da sich die Partikel nach 8-10 h normalerweise im Dickdarm befinden, wo sowohl die Resorptionsoberfläche als auch das für die Freisetzung benötigte Wasser deutlich reduziert sind.

Ebenso bevorzugt sind Ausführungen, welche nach 4 Stunden zwischen 50 % und 80 % des Wirkstoffs freigesetzt haben, und/oder nach 6 Stunden zwischen 60 % und 90 %, und/oder nach 8 Stunden zwischen 65 % und 100 %.

Um die gewünschte Retardierung über ca. 6-8 h und/oder von nicht mehr als 85 % innerhalb von 4 Stunden mit den Retardpartikeln zu erzielen, sollte das Gewichtsverhältnis zwischen Kern und Lipidüberzug zwischen 60:40 und 90:10 liegen. Vorteilhaft ist auch ein Gewichtsverhältnis zwischen 80:20 und 95:5, z. Bsp. 85:15 oder 90:10. Hierbei sind Gewichtsverhältnisse oberhalb von 80:20, wie bspw. 85:15, 90:10 oder 95:5, auch deshalb vorteilhaft, weil geringere Auftragsmengen des Überzugsmaterials typischerweise mit kürzeren Prozesszeiten einhergehen.

Während der Überzug der Retardpartikel vorrangig die gewünschte retardierte Freisetzung des Wirkstoffs sicherstellt, fungiert dieser aber auch als Geschmacksmaskierung für die in den Kernen enthaltene Magnesium-verbindung, da diese aufgrund der Retardierung kaum oder gar nicht auf der Zunge freigesetzt wird. Gerade für einige der sehr sauer oder geschmacklich wenig akzeptabel schmeckenden Magnesiumverbindungen, wie bspw. einigen Magnesium-Citraten oder Magnesium-Acetat, kann dies unter Umständen vorteilhaft sein.

Erfindungsgemäß ist die Zusammensetzung in Form eines oralen Direktgranulats so aufgebaut, dass sie neben den überzogenen Magnesium-Retardpartikeln der Komponente (a) auch einen Anteil nicht-retardierter Magnesium-haltige Partikel als Komponente (b) enthalten. Diese sind vorrangig als schnell-freisetzende Initialdosis gedacht. Üblicherweise ist die Menge bzw. Dosis an nicht-retardiertem Magnesium kleiner als die der überzogenen Magnesium-Retardpartikeln; bspw. etwa 5-100 mg nicht-retardiertes Magnesium in Kombination mit Dosis 50-700 mg Magnesium-Retardpartikeln, z. Bsp. ca. 8 mg Initialdosis plus 500 mg retardiertes Magnesium, oder ca. 50 mg Initialdosis plus 350 mg retardiertes Magnesium. Die beiden Teildosen sollten hierbei so gewählt sein, dass sie in der Summe eine Tagesdosis von ca. 800 mg Magnesium, bevorzugt ca. 600 mg, nach Möglichkeit nicht überschreiten.

In einer Ausführungsform ist die Magnesiumverbindung in den nicht-retardierten Partikeln der Komponente (b) ausgewählt aus Magnesiumoxid (MgO), Magnesiumcarbonat (MgCO₃), Magnesiumacetat (Mg(CH₃COO)₂), Magnesiumhydrogenphosphat (MgHPO₄) Magnesiumhydrogencitrat, Trimagnesiumdicitrat (C₁₂H₁₀Mg₃O₁₄), Magnesiumpidolat (C₁₀H₁₂MgN₂O₆), Magnesium N-Acetyltaurinat (C₈H₁₆MgN₂O₈S₂) und/oder Magnesium-bis(hydrogen-L-glutamat), sowie deren Hydraten. In einer spezifischen Ausführungsform ist die Magnesiumverbindung in den nicht-retardierten Partikeln der Komponente (b) ausgewählt aus Magnesiumhydrogencitrat und/oder Trimagnesiumdicitrat. In einer spezifischeren Ausführungsform bestehen die nicht-retardierten Partikel der Komponente (b) im Wesentlichen aus Magnesium-hydrogencitrat und/oder Trimagnesiumdicitrat.

In einer Ausführungsform enthalten die Retardpartikel der Komponente (a) eine andere Magnesiumverbindung als die nicht-retardierten Partikel der Komponente (b). So kann bspw. in einer der bevorzugten Ausführungsformen der Kern der Retardpartikel Magnesiumoxid enthalten, und die nicht-retardierten Partikel der Komponente (b) enthalten Magnesiumhydrogencitrat und/oder Trimagnesiumdicitrat. Weiterhin bevorzugt sind Ausführungsformen, in denen der Kern der Retardpartikel im Wesentlichen aus Magnesiumoxid besteht, und die nicht-retardierten Partikel der Komponente (b) im Wesentlichen aus Magnesiumhydrogencitrat und/oder Trimagnesiumdicitrat bestehen.

Trimagnesiumdicitrat ist ein nicht-saures Salz der Zitronensäure, und kann somit geschmacklich neutraler sein als bspw. Magnesiumhydrogencitrat, was unter Umständen für ein orales Direktgranulat vorteilhaft sein kann.

In einer Ausführungsform weisen die Retardpartikel der Komponente (a), und optional die nicht-retardierten Partikel der Komponente (b), eine mediane Teilchengröße (D50), gemessen mittels dynamischer Bildanalyse (z. Bsp. mittels Camsizer^{®} XT), von ≤ 500 µm auf, bevorzugt ≤ 450 µm, weiter bevorzugt ≤ 400 µm. Wie oben erwähnt, sollte die Partikelgröße von oralen Direktgranulaten im Allgemeinen 600 µm, bevorzugt 500 µm, nicht überschreiten, um ein Fremdkörpergefühl im Mund zu vermeiden und den Kaureflex gering zu halten. Mit anderen Worten, die Mehrzahl der Partikel (mindestens 80 %, bevorzugt mindestens 90 %.) sollte ein Sieb mit einer Maschenweite von ca. 600 µm passieren. In einer bevorzugten Ausführungsform der Erfindung haben daher die Retardpartikel der Komponente (a) - und optional die nicht-retardierten Partikel der Komponente (b) - eine mediane Teilchengröße (D50), gemessen mittels dynamischer Bildanalyse, zwischen 100 µm und 500 µm, bevorzugt zwischen 150 µm und 450 µm, weiterhin bevorzugt zwischen 200 µm und 400 µm.

In einer Ausführungsform machen die Retardpartikel der Komponente (a) zwischen 15 und 85 Gew.-%, oder zwischen 25 und 75 Gew.-%, oder zwischen 30 und 70 Gew.-%, oder zwischen 30 und 60 Gew.-% der festen pharmazeutischen oder nutrazeutischen Zusammensetzung gemäß dem ersten Aspekt aus; bspw. 30-35 Gew.-% oder 55-60 Gew.-%. Die Wahl ist hierbei vorrangig davon geleitet, welcher Anteil der Magnesium-Gesamtdosis in der Zusammensetzung retardiert freigegeben werden soll und wie hoch die Einmaldosis für die Applikation gewählt wird.

Neben den Retardpartikeln der Komponente (a) und den nicht-retardierten Magnesium-haltigen Partikeln der Komponente (b) enthält die Zusammensetzung in Form eines oralen Direktgranulats mindestens einen wasserlöslichen Hilfsstoff gemäß Komponente (c), mit dem die anderen Komponenten vermischt werden. Es handelt sich hierbei erfindungsgemäß um Hilfsstoffe, die gemäß Definition des Europäischen Arzneibuchs löslich, leicht löslich oder sehr leicht löslich sind in Wasser (d.h. 1 g zu lösender Substanz benötigt nicht mehr als 30 mL Wasser) und die aus der Gruppe der Zucker, Zuckeralkohole und Oligosaccharide ausgewählt sind. Bei der Verabreichung des oralen Direktgranulates fungieren die wasserlösliche Hilfsstoffe der Komponente (c) quasi als äußere Phase einer schluckbaren, in-situ gebildeten Suspension, mit deren Hilfe die Retardpartikel leichter geschluckt werden können. Bevorzugt werden daher schmackhafte, wasserlösliche Hilfsstoffe wie Zucker, Zuckeralkohole oder wasserlösliche Vertreter der Oligosaccharide gewählt. Dies unterstützt auch die Compliance bei einer dauerhaften Anwendung des Direktgranulates. Zwar wäre es theoretisch möglich, die Retardpartikel der Komponente (a) und die Magnesiumhaltigen Partikel der Komponente (b) auch allein, ohne weitere Hilfsstoffe zu verabreichen, allerdings würden diese sich in dem Fall nicht ganz so leicht und angenehm schlucken lassen, länger im Mund verbleiben und vom Anwender möglicherweise zerkaut werden, wodurch der Retard-Effekt aufgehoben würde.

Die Menge an Hilfsstoffen, insbesondere die Menge der Komponenten (c) und (d), sollte hierbei vorzugsweise so gewählt sein, dass die Gesamtmenge an oralem Direktgranulat pro Dosis nicht mehr als ca. 3 g beträgt, oder nicht mehr als etwa 2,5 g oder 2,0 g, da größere Pulver- bzw. Granulat-Mengen im Mund und auf der Zunge nur noch schwierig gleichmäßig zur durchfeuchten sind. Sofern nötig kann eine Einzeldosis in zwei oder mehreren Teilen (z. Bsp. 2-3 Sachets mit nicht mehr als ca. 3 g, bevorzugt nicht mehr als ca. 2 g, Direktgranulat) eingenommen werden.

In einer Ausführungsform enthält die feste pharmazeutische oder nutrazeutische Zusammensetzung gemäß dem ersten Aspekt mindestens 20 Gew.-%, bevorzugt mindestens 23 Gew.-%, weiterhin bevorzugt mindestens 25 Gew.-% der Hilfsstoffe gemäß Komponente (c).

In einer Ausführungsform enthalten die Hilfsstoffe der Komponente (c) in der festen pharmazeutischen oder nutrazeutischen Zusammensetzung gemäß dem ersten Aspekt mindestens 60 Gew.-%, bevorzugt mindestens 75 Gew.-%, weiterhin bevorzugt mindestens 85 Gew.-%, eines in Wasser löslichen Zuckers oder Zuckeralkohols; optional bestehen sie im Wesentlichen hieraus. Hierbei kann der in Wasser lösliche Zucker oder Zuckeralkohol ausgewählt sein aus Saccharose, Sorbitol, Xylitol, Erythritol, Maltitol, Isomaltitol, Mannitol, und Mischungen derselben. In einer spezifischen Ausführungsform sind die oralen Direktgranulate so zusammengesetzt sind, dass die Hilfsstoffe der Komponenten (c) und (d) mindestens 50 Gew.-%, oder mindestens 55 Gew.-% Sorbitol oder Xylitol enthalten, oder im Falle einer Mischung aus Sorbitol und Xylitol mindestens 50 Gew.-%, oder mindestens 55 Gew.-%, dieser Mischung. In einzelnen Ausführungsformen können die oralen Direktgranulate auch so zusammengesetzt sein, dass die Hilfsstoffe der Komponenten (c) und (d) sogar mindestens 85 Gew.-%, oder mindestens 90 Gew.-% Sorbitol oder Xylitol enthalten, oder im Falle einer Mischung aus Sorbitol und Xylitol mindestens 85 Gew.-%, oder mindestens 90 Gew.-%, dieser Mischung.

Durch diese Gewichtsanteile der löslichen Zucker, Zuckeralkohole oder Oligosaccharide wird ein ausreichendes Volumen an flüssigem wohlschmeckendem Suspensionsmedium für die erfindungsgemäße Zusammensetzung im Mund des Anwenders gebildet und die Schluckbarkeit sowie die Compliance des Anwenders positiv beeinflusst.

Optional können der Zusammensetzung in Form eines oralen Direktgranulats neben den Zuckern, Zuckeralkoholen und/oder Oligosacchariden noch weitere Hilfsstoffe gemäß der Komponente (d) zugesetzt sein, die beispielsweise die Viskosität der sich in-situ bildenden Granulat-Suspension auf der Zunge leicht erhöhen, die den Speichelfluss anregen, oder die den Geschmack beeinflussen oder die Riesel- bzw. Fließfähigkeit des Granulates regulieren. Optional können auch die Hilfsstoffe gemäß (d) wasserlöslich sein (also löslich, leicht löslich oder sehr leicht löslich gemäß der Definition des Europäischen Arzneibuchs).

In einer Ausführungsform enthält die feste pharmazeutische oder nutrazeutische Zusammensetzung gemäß dem ersten Aspekt beispielsweise einen sauren Hilfsstoff als Bestandteil der Hilfsstoffe gemäß Komponente (d). Bevorzugte saure Hilfsstoffe sind Zitronensäure, Weinsäure, Äpfelsäure, Bernsteinsäure, sowie saure Salze derselben, etwa Mononatriumcitrat. Die Erfinder haben festgestellt, dass der Einsatz dieser sauren Substanzen - vor allem in Kombination mit den löslichen Zuckern und Zuckeralkoholen in den bevorzugten Mengen - eine zusätzliche Anregung des Speichelflusses hervorruft, durch den die Zusammensetzung, und insbesondere die typischerweise etwas größeren Retardpartikel darin, optimal dispergiert werden und besonders leicht zu schlucken sind. In einer spezifischen Ausführungsform enthält die Zusammensetzung als weitere Hilfsstoffe gemäß Komponente (d) Zitronensäure, Mononatriumcitrat und/oder Dinatriumcitrat, insbesondere Zitronensäure in Kombination mit Mononatriumcitrat.

In einer weiteren Ausführungsform kann die Zusammensetzung in Form oraler Direktgranulate so zusammengesetzt sein, dass die Hilfsstoffe der Komponente (d) viskositätserhöhende Substanzen als schluckbarkeitsfördernde Hilfsstoffe enthalten, z. Bsp. Celluloseether wie Carmellose-Natrium (auch Natrium-Carboxymethylcellulose genannt; typischerweise in der nicht quervernetzten Form) oder modifizierte Stärken wie bspw. Lycatab^{®} PGS (eine vollständig vorverkleisterte Maisstärke). Ihre Konzentration sollte so gewählt werden, dass sie einerseits das Mundgefühl des Direktgranulates verbessern, und die in situ daraus entstehende Retardpartikel-Suspension stabilisieren, andererseits aber das Schlucken nicht erschweren; sprich die auf der Zunge geformte Suspension sollte sämig sein, nicht schleimig oder klebrig.

Weiter optional können die oralen Direktgranulate so zusammengesetzt sein, dass die Hilfsstoffe der Komponente (d) Gleitmittel enthalten; z. Bsp. dem Fachmann bekannte Fließregulierungs- und Schmiermittel wie hochdisperses Siliciumdioxid, Magnesiumstearat und/oder Stearinsäure. Diese Substanzen dienen dazu, das orale Direktgranulat streufähig bzw. fließ- oder rieselfähig zu machen, so dass es leicht abgefüllt und auch leicht wieder aus Verpackungen entnommen werden kann. Magnesiumstearat wird hierbei bevorzugt, wird aber explizit nicht mit zu den Magnesium-haltigen Komponenten (a) oder (b) gezählt, da Magnesiumstearat, sofern verwendet, nur in vergleichsweise geringen Mengen zum Einsatz kommt (≤0,50 Gew.-%, bevorzugt ≤0,30 Gew.-%, weiter bevorzugt ≤0,25 Gew.-%), um so einen wachsartigen Eindruck im Mund zu vermeiden.

Des Weiteren können Aromen, Süßstoffe, Geschmackskorrigenzien, Farbstoffe usw. als Bestandteil der Hilfsstoffe gemäß Komponente (d) verwendet werden, um die organoleptischen Eigenschaften der erfindungsgemäßen Zusammensetzung anzupassen. So können die oralen Direktgranulate bspw. so zusammengesetzt sein, dass die Hilfsstoffe neben den löslichen Zuckern, Zuckeralkoholen oder Oligosacchariden der Komponente (c) weitere Süßungsmittel bzw. Süßstoffe (bspw. Aspartam, Acesulfam-K, Sucralose oder dgl.), und/oder Aromen (Zitrone, Orange, Tropenfrüchte, etc.) enthalten. Gängige und verträgliche Süßungsmittel und Aromen sind dem Fachmann hinreichend bekannt, z. Bsp. aus oralen pädiatrischen Formulierungen.

Optional können die oralen Direktgranulate zusätzlich zu den Komponenten (a) bis (d), und insbesondere zusätzlich zum Magnesium, noch weitere nutrazeutische Substanzen enthalten, wie z. Bsp. Mineralien und/oder Vitamine. Optional können auch diese in retardierter Form vorliegen, bspw. mit retardierenden Überzügen und/oder eingebettet in eine retardierende Matrix. In einer Ausführungsform enthält die Zusammensetzung bspw. Vitamine der B-Reihe und/oder Vitamin D. In einer spezifi-schen Ausführungsform enthält die Zusammensetzung bspw. Magnesium in Kombination mit Vitamin D3 und B12. In einer weiteren spezifischen Ausführungsform enthält die Zusammensetzung bspw. Magnesium in Kombination mit Vitamin B1, B2, B6 und B12.

In einer Ausführungsform sind die Mengen der Retardpartikel der Komponente (a) und der nicht-retardierten Partikel der Komponente (b) so gewählt, dass innerhalb von 4 Stunden, bevorzugt innerhalb von 5 Stunden, weiter bevorzugt innerhalb von 6 Stunden, nicht mehr als 85 % des in der Zusammensetzung enthaltenen Magnesiums freisetzen, gemessen in einer Freisetzungs-Apparatur mit Blattrührer (USP Dissolution Apparatus 2) in 900 mL 0.1 N Salzsäure bei 37 °C und einer Rührgeschwindigkeit von 75 UPM. Um dies zu gewährleisten sind typischerweise mindestens 60 Gew.-%, oder mindestens 75 Gew.-%, oder mindestens 85 Gew.-%, des gesamten in der Zusammensetzung enthaltenen Magnesiums in den Retardpartikeln der Komponente (a) enthalten. Alternativ oder zusätzlich hierzu enthält die Zusammensetzung typischerweise mindestens 5 Gew.-%, oder mindestens 10 Gew.-%, oder mindestens 12 Gew.-%, und bis maximal 25 Gew.-%, des gesamten in der Zusammensetzung enthaltenen Magnesiums in nicht-retardierter Form.

Optional können einzelne oder alle der Substanzen oder Hilfsstoffe gemäß Komponenten (b), (c) und/oder (d), welche den Retardpartikeln der Komponente (a) beigemischt werden, in granulierter Form vorliegen. Dies kann z. Bsp. in Fällen vorteilhaft sein, um einen potenziell störenden Feinstaubanteil zu reduzieren und/oder wenn die Mischung aus den Retardpartikeln der Komponente (a) und die Komponenten gemäß (b), (c) und/oder (d) zur Entmischung neigen (z. Bsp., wenn die Partikelgröße(n) aller oder einzelner Stoffe in den Komponenten (b), (c) und/oder (d) deutlich unter der Partikelgröße der Retardpartikel liegen). Eine Angleichung oder Annäherung der Partikelgrößen der Komponenten (b), (c) und/oder (d) an die Partikelgröße der Retardpartikel der Komponente (a) durch Granulierung kann diesen Entmischungstendenzen entgegenwirken und so ein gleichmäßiges Ausgießen des oralen Direktgranulats gewährleisten. In einer Ausführungsform weisen auch die beigemengten Hilfsstoffpartikel der Komponenten (b), (c) und/oder (d), eine mediane Teilchengröße (D50), gemessen mittels dynamischer Bildanalyse, zwischen 100 µm und 500 µm auf, bevorzugt zwischen 150 µm und 450 µm, weiterhin bevorzugt zwischen 200 µm und 400 µm.

In einer der bevorzugten Ausführungsformen ist das orale Direktgranulat zudem so zusammengesetzt, dass die Hilfsstoffe der Komponenten (c) und (d) nicht mehr als 5 Gew.-% von in Wasser schwerlöslichen Hilfsstoffen enthalten; d.h. umgekehrt sollten rund 95 Gew.-% oder mehr der Hilfsstoffe löslich sein. Dies ist wünschenswert, um das Fremdkörpergefühl im Mund so gering wie möglich zu halten.

In einer der bevorzugten Ausführungsformen sind Einzeldosen der Zusammensetzung in Stickpacks, Sachets, Glasampullen oder Plastikampullen verpackt, um dem Anwender so die Dosierung des Magnesiums zu erleichtern. Stickpacks sind hierbei besonders bevorzugt, da sie leicht und bruchsicher transportiert werden können und auch beim Öffnen durch Aufreißen üblicherweise eine kleinere Schüttöffnung bieten - und somit leichter vollständig in den Mund geleert werden können - als die vergleichbar aufgebauten, aber meist breiter dimensionierten Sachets. Zudem bieten Stickpacks aufgrund ihrer Laminierung eine einfache Möglichkeit zum Schutz des Direktgranulates vor Licht und Sauerstoff und stabilisieren so das in der Zusammensetzung enthaltene Magnesium zusätzlich.

In einer Ausführungsform enthalten die Einzeldosen der Zusammensetzung typischerweise zwischen 50 und 700 mg Magnesium, bevorzugt zwischen 100 und 650 mg, weiter bevorzugt zwischen 150 und 600 mg oder zwischen 350 und 550 mg, z. Bsp. ca. 400 mg, ca. 500 mg oder ca. 510 mg. In einer spezifischen Ausführungsform enthält eine Einzeldosis der Zusammensetzung ca. 350 mg Magnesium in den Retardpartikeln der Komponente (a) und ca. 50 mg Magnesium in den nicht-retardierten Partikeln der Komponente (b); bspw. ca. 350 mg Magnesium in Form von Magnesiumoxid in den Retardpartikeln und ca. 50 mg Magnesium in Form von nicht-retardiertem Magnesiumhydrogencitrat, oder alternativ ca. 350 mg Magnesium in Form von Magnesiumoxid gemischt mit ca. 42 mg Magnesium in Form von nicht-retardiertem Magnesiumhydrogencitrat und ca. 8 mg Magnesium in Form von nicht-retardiertem Trimagnesiumdicitrat. In einer weiteren spezifischen Ausführungsform enthält eine Einzeldosis der Zusammensetzung ca. 510 mg Magnesium in den Retardpartikeln der Komponente (a) und ca. 8 mg Magnesium in den nicht-retardierten Partikeln der Komponente (b); bspw. ca. 500 mg Magnesium in Form von Magnesiumoxid und ca. 10 mg Magnesium in Form von nicht-retardiertem Trimagnesiumdicitrat.

In einer der bevorzugten Ausführungsformen wird eine Zusammensetzung in Form eines oralen Direktgranulats bereitgestellt, enthaltend:
Retardpartikel mit einem Magnesium-haltigen Kern und einem Überzug welcher zu mindestens 90 Gew.-% aus Glyceroltripalmitat und/oder Glyceroltristearat besteht,
bspw. Dynasan^{®}116 oder 118, wobei das Gewichtsverhältnis zwischen Kern und Überzug zwischen 70:30 und 90:10 liegt;
nicht-retardierte Magnesium-Partikel;
mindestens einen oder mehrere wasserlösliche Hilfsstoffe ausgewählt aus Saccharose, Sorbitol, Xylitol, Erythritol, Maltitol, Isomaltitol, Mannitol und Mischungen derselben; mindestens einen oder mehrere speichelanregende Hilfsstoffe, ausgewählt aus Zitronensäure, Mononatriumcitrat, Dinatriumcitrat und Mischungen derselben;
optional einen oder mehrere schluckbarkeitsfördernde Hilfsstoffe ausgewählt aus Celluloseethern oder modifizierten Stärken und Mischungen derselben;
weiterhin optional ein Gleitmittel ausgewählt aus Magnesiumstearat, Stearinsäure und hochdispersem Siliciumdioxid oder Mischungen derselben; mindestens ein Aroma; sowie optional weitere Süßstoffe oder Süßungsmittel, wobei der Gewichtsanteil der Retardpartikel im oralen Direktgranulat zwischen 25 % und 75 % liegt.

In einer weiteren bevorzugten Ausführungsformen wird eine Zusammensetzung in Form eines oralen Direktgranulats bereitgestellt, enthaltend:
Retardpartikel mit einem Magnesium-haltigen Kern enthaltend, oder bestehend aus, Magnesiumoxid, Magnesiumcarbonat, oder Magnesiumchlorid, und einem Überzug welcher zu mindestens 90 Gew.-% aus Glyceroltripalmitat und/oder Glyceroltristearat besteht, bspw. Dynasan^{®}116 oder 118, wobei das Gewichtsverhältnis zwischen Kern und
Überzug zwischen 80:20 und 90:10 liegt;
nicht-retardierte Magnesium-Partikel enthaltend, oder bestehend aus, Magnesiumhydrogencitrat und/oder Trimagnesiumdicitrat;
mindestens einen oder mehrere wasserlösliche Hilfsstoffe ausgewählt aus Sorbitol, Xylitol und Mischungen derselben;
mindestens einen oder mehrere speichelanregende Hilfsstoffe, ausgewählt aus Zitronensäure, Mononatriumcitrat und Mischungen derselben;
optional einen oder mehrere schluckbarkeitsfördernde Hilfsstoffe ausgewählt aus Natrium-Carboxymethylcellulose, vollständig vorverkleisterter Stärke (bspw. Maisstärke) und Mischungen derselben;
weiterhin optional Magnesiumstearat als Gleitmittel;
mindestens ein Aroma; sowie mindestens einen Süßstoff ausgewählt aus Sucralose, Aspartam, Acesulfam-K und Mischungen derselben, wobei der Gewichtsanteil der Retardpartikel im oralen Direktgranulat zwischen 30 % und 70 % liegt.

Der Vorteil der erfindungsgemäßen Zusammensetzung in Form eines oralen Direktgranulates gemäß dem ersten Aspekt der Erfindung liegt darin, dass es üblicherweise ohne zusätzliche Flüssigkeit, allein mittels des im Mund vorhandenen Speichel durchfeuchtet bzw. suspendiert und anschließend leicht und angenehm geschluckt werden kann, dabei aber gleichzeitig die kontrollierte Freisetzung des Magnesiums in Form einer retardierten Dosis in Kombination mit einer nicht-retardierten Dosis sicherstellt, selbst wenn in Summe höhere Dosen von z. Bsp. 400 mg Magnesium oder mehr vorliegen. Letzteres gelingt sonst häufig nur in Form voluminöser, schwer schluckbarer Kapsel- oder Tablettenformulierungen.

Diese vorteilhafte Schluckbarkeit wird u.a. durch eine fein aufeinander abgestimmte Selektion und Kombination von Parametern erreicht bzw. unterstützt, wie z. Bsp. die Art und Menge der Hilfsstoffe, welche den Retardpartikeln beigemengt werden, der Gewichtsanteil der Retardpartikel im Direktgranulat, ihre Partikelgröße (sowie die Partikelgröße weiterer Bestandteile des Direktgranulats), die Wahl der Überzugsmaterialien, etc. Vorteilhafte Ausführungsformen sind oben beschrieben.

Die vorteilhafte Verarbeitbarkeit des retardierten Dosis-Anteils in den Retardpartikeln wiederum wird u.a. durch die spezifische Selektion von Magnesium-Kernen mit den beanspruchten Parametern erreicht, welche das Auftragen des Schmelzüberzugs überhaupt erst ermöglichen, insbesondere im Industriemaßstab.

In einem zweiten Aspekt betrifft die vorliegende Erfindung ein Verfahren zur Herstellung einer festen pharmazeutischen oder nutrazeutischen Zusammensetzung gemäß dem oben beschriebenen ersten Aspekt der Erfindung, wobei das Verfahren die folgenden Schritte enthält bzw. umfasst:
(i) Bereitstellen eines Magnesium-haltigen Kerns, wobei der Kern eine Magnesium-verbindung enthält oder aus dieser besteht;
(ii) Bereitstellen eines geschmolzenen Überzugsmaterials enthaltend ein Lipid;
(iii) Fluidisieren der Magnesium-haltigen Kerne;
(iv) Besprühen der fluidisierten Magnesium-haltigen Kerne mit dem geschmolzenen Überzugsmaterial;
(v) Abkühlen der überzogenen Magnesium-haltigen Kerne, so dass das Lipid erstarrt und Retardpartikel gemäß Komponente (a) erhalten werden, welche das enthaltene Magnesium in retardierter Weise freisetzen; und
(vi) Mischen der so als Komponente (a) erhaltenen Retardpartikel mit den folgenden weiteren Komponenten:
   (b) nicht-retardierte Partikel mit einem zweiten Magnesium-haltigen Kern, wobei der Partikel eine Magnesiumverbindung enthält oder aus dieser besteht, und wobei die Partikel das enthaltene Magnesium in nicht-retardierter Weise freisetzen;
   (c) einen oder mehrere wasserlösliche Hilfsstoffe ausgewählt aus der Gruppe der Zucker, Zuckeralkohole und Oligosaccharide; und
   (d) optional weiteren Hilfsstoffen;
      dadurch gekennzeichnet, dass mindestens die Magnesium-haltigen Kerne der Retardpartikel der Komponente (a) eine solche Abriebfestigkeit aufweisen, dass nach einer Vibrationssiebung über einen Zeitraum von 60 Minuten:
      - der Feinanteil an Partikeln mit einem Siebdurchmesser von < 200 µm nach einer Siebzeit von 60 Minuten, t₆₀ₘᵢₙ, um maximal 100 Gew.-% höher ist gegenüber dem initialen Feinanteil dieses Siebdurchmessers nach einer Siebzeit von 5 Minuten, t₅ₘᵢₙ; und/oder
      - der Anteil an Partikeln mit einem Siebdurchmesser von 200 µm ≤ x < 800 µm nach einer Siebzeit von 60 Minuten, t₆₀ₘᵢₙ, um maximal 18 Gew.-% niedriger ist gegenüber dem initialen Anteil dieses Siebdurchmessers nach einer Siebzeit von 5 Minuten, t₅ₘᵢₙ.

Alle Ausführungsformen, die in Verbindung mit der pharmazeutischen oder nutrazeutischen Zusammensetzung gemäß dem ersten Aspekt der Erfindung offenbart sind, einschließlich bevorzugte Ausführungsformen, können gleichermaßen auf das Herstellverfahren gemäß dem zweiten Aspekt der Erfindung angewendet werden.

So weisen bspw. in einer spezifischen Ausführungsform mindestens die im Schritt (i) bereitgestellten Magnesium-haltigen Kerne der Retardpartikel der Komponente (a) eine solche Abriebfestigkeit auf, dass nach einer Vibrationssiebung über einen Zeitraum von 60 Minuten der Feinanteil an Partikeln mit einem Siebdurchmesser von < 200 µm nach einer Siebzeit von 60 Minuten, t₆₀ₘᵢₙ, um maximal 50 Gew.-%, bevorzugt um maximal 20 Gew.-%, höher ist gegenüber dem initialen Feinanteil dieses Siebdurchmessers nach einer Siebzeit von 5 Minuten, t₅ₘᵢₙ; und/oder der Anteil an Partikeln mit einem Siebdurchmesser von 200 µm ≤ x < 800 µm nach einer Siebzeit von 60 Minuten, t₆₀ₘᵢₙ, um maximal 15 Gew.-%, bevorzugt um maximal 12 Gew.-%, niedriger ist gegenüber dem initialen Anteil dieses Siebdurchmessers nach einer Siebzeit von 5 Minuten, t₅ₘᵢₙ.

In einer weiteren Ausführungsform weisen mindestens die im Schritt (i) bereitgestellten Magnesium-haltigen Kerne der Retardpartikel der Komponente (a) eine solche Abriebfestigkeit auf, dass nach einer Vibrationssiebung über einen Zeitraum von 60 Minuten der Anteil an Partikeln mit einem Siebdurchmesser von < 300 µm nach einer Siebzeit von 60 Minuten, t₆₀ₘᵢₙ, um maximal 55 Gew.-%, bevorzugt um maximal 35 Gew.-%, weiter bevorzugt um maximal 15 Gew.-%, höher ist gegenüber dem initialen Anteil dieses Siebdurchmessers nach einer Siebzeit von 5 Minuten, t₅ₘᵢₙ; und/oder der Anteil an Partikeln mit einem Siebdurchmesser von 300 µm ≤ x < 800 µm nach einer Siebzeit von 60 Minuten, t₆₀ₘᵢₙ, um maximal 30 Gew.-%, bevorzugt um maximal 27 Gew.-%, weiter bevorzugt um maximal 24 Gew.-%, niedriger ist gegenüber dem initialen Anteil dieses Siebdurchmessers nach einer Siebzeit von 5 Minuten, t₅ₘᵢₙ.

Die Vibrationssiebung kann wie oben bereits beschrieben durchgeführt werden: eine zu siebende Menge von ca. 100 g der Magnesium-haltigen Kerne wird auf den Siebturm gegeben, zusammen mit je ca. 1 g Siebhilfe pro Siebboden (hier abriebfeste 2-5 mm Silicagel-Kugeln mit einer Schüttdichte von ca. 750 kg/m³) und für 60 min bei einer Vibrationsamplitude von 80/min gesiebt und auf diese Weise mechanisch gestresst.

In einer der vorteilhaften Ausführungsformen ist die Siebanalyse dem Verfahren gemäß dem zweiten Aspekt der Erfindung vorangestellt, um so vorab die Tauglichkeit der Magnesium-haltigen Kerne für ein Schmelzbeschichtungsverfahren (Hot-Melt Coating) in der Wirbelschicht zu prüfen.

Kerne, welche zu viel Abrieb aufweisen (sprich mehr als im Rahmen der Erfindung zulässig), eignen sich nicht für ein Hot-Melt Coating in der Wirbelschicht, insbesondere nicht im Industriemaßstab. Während früherer Versuche, ein schmelzüberzogenes Magnesium-Direktgranulat herzustellen, hatten die Erfinder festgestellt, dass der starke Abrieb einiger Magnesium-Qualitäten, die Filter zu stark verstopfte weit bevor der Schmelzüberzugsprozess ausreichend weit vorangeschritten war; ein Problem, dass sich auch nicht mit den üblichen automatisierten Filterabschlägen der Wirbelschichtgeräte beheben ließ. Die somit in diesen Versuchen benötigten häufigen Filterwechsel, bzw. Filterreinigungsschritte, machten die früheren Verfahren ungeeignet für den Industriemaßstab. Zudem waren, wie oben erwähnt, die entstehenden Auflagerungen von verkrustetem Kern-Abrieb und Sprühmaterial auf den Innenwänden des Wirbelschicht-Geräts so ausgeprägt und fest, dass stets sehr zeitaufwändige Reinigungsschritte nötig waren.

Das in den Schritten (i)-(v) beschriebene Verfahren, und optional auch der Mischungsschritt (vi), kann in Wirbelschichtgeräten (sog. fluid-bed coater oder air-flow-bed coater) jeglicher Art durchgeführt werden, sofern die Geräte eine ausreichende Temperierung erlauben, um eine vorzeitige Erstarrung des geschmolzenen Überzugsmaterials zu vermeiden. Temperatur und Sprührate der Schmelze sollte in jedem Fall so angepasst sein, dass eine gleichmäßige Befilmung der einzelnen Kern-Partikel gewährleistet ist, da größere Agglomerate, in denen bspw. zwei oder mehr Kern-Partikel miteinander ,verklebt' sind, für ein gutes Mundgefühl des Direktgranulates nicht erwünscht sind.

Wie oben erwähnt, enthält der Überzug der Retardpartikel in einer der bevorzugten Ausführungsformen ein Lipid mit einem Schmelzpunkt von mindestens 50 °C, oder besteht im Wesentlichen hieraus; bevorzugt ein Lipid mit einem Schmelzpunkt von mindestens 60 °C. Um einen gleichmäßigen Auftrag des geschmolzenen Überzugsmaterials zu gewährleisten, liegt in einer Ausführungsform des Verfahrens die Prozesslufttemperatur in den Schritten (iii) und/oder (iv) zwischen 20 und 60 °C, oder zwischen 20 und 50 °C, oder zwischen 20 und 45 °C; bspw. bei ca. 25 °C oder 35 °C.

In einer Ausführungsform des Verfahrens wird das in Schritt (ii) bereitgestellte geschmolzene Überzugsmaterial im Schritt (iv) bei einer Schmelztemperatur zwischen 70 und 120 °C, oder zwischen 75 und 110 °C, oder zwischen 80 und 100 °C versprüht; bspw. bei ca. 90 bis 100 °C. So liegt bspw. der Schmelzpunkt von Glyceroltristearat (z. Bsp. Dynasan^{®}118) bei ca. 70-73 °C; daher sollte die Temperatur der zu sprühenden Schmelze hierbei einige Grad darüber liegen; z. Bsp. bei 90 °C.

In einer Ausführungsform des Verfahrens wird im Schritt (iv) eine Sprühlufttemperatur zwischen 70 und 130 °C, oder zwischen 75 und 125 °C, oder zwischen 80 und 120 °C verwendet; bspw. ca. 100 °C oder 120 °C. Des Weiteren wird in einer Ausführungsform des Verfahrens im Schritt (iv) ein Sprühdruck zwischen 0,5 und 1,5 bar gewählt, oder zwischen 0,6 und 1,3 bar, oder zwischen 0,7 und 1,2 bar; bspw. bei 0,8 bis 1,2 bar, 0,8 bis 0,9 bar oder 1,0 bis 1,2 bar.

In einer Ausführungsform des Verfahrens liegt die Sprühmenge des geschmolzenen Überzugsmaterials pro Kilogramm zu überziehendem Kern-Material im Schritt (iv) zwischen 2,0 und 10,0 g/kg/min, oder zwischen 2,5 und 9,0 g/kg/min, oder zwischen 3,0 und 8,0 g/kg/min; bspw. bei ca. 3,3 g/kg/min. In einer spezifischen Ausführungsform des Verfahrens wird dieses - und insbesondere die Überzugsschritte (iii) bis (v) - in einem Wirbelschichtgerät im Labor-Maßstab (z. Bsp. in einem Ventilus^{®}V-2.5) durchgeführt, und die Sprühmenge des geschmolzenen Überzugsmaterials im Schritt (iv) liegt zwischen 5,0 und 8,0 g/min, oder zwischen 5,5 und 7,5 g/min, oder zwischen 6,0 und 7,0 g/min; bspw. bei ca. 6,5 g/min.

Die gemäß Verfahrensschritt (vi) neben den Retardpartikeln der Komponente (a) vorliegenden Komponenten (c) bis (d), optional (b) bis (d), werden üblicherweise zuerst homogen miteinander vermischt, bevor sie anschließend mit den Retardpartikeln vermengt werden. Wie erwähnt können optional einzelne oder alle der Substanzen oder Hilfsstoffe gemäß Komponenten (b), (c) und/oder (d) vor dem Vermengen mit den Retardpartikeln granuliert bzw. aggregiert werden.

In einer Ausführungsform des Verfahrens werden in einem weiteren Schritt, im Anschluss an Schritt (vi), Einzeldosen der Zusammensetzung in Stickpacks, Sachets, Glasampullen oder Plastikampullen verpackt.

In einem weiteren Aspekt der Erfindung betrifft die vorliegende Erfindung eine feste pharmazeutische oder nutrazeutische Zusammensetzung in Form eines oralen Direktgranulats mit mindestens dualer Wirkstofffreisetzung, welche(s) mittels des Verfahrens gemäß dem zweiten Aspekt der Erfindung hergestellt wird.

Die folgende Liste stellt Ausführungsformen vor, welche von der vorliegenden Erfindung umfasst sind:
1. Feste pharmazeutische oder nutrazeutische Zusammensetzung in Form eines oralen Direktgranulats mit mindestens dualer Wirkstofffreisetzung, enthaltend folgende Komponenten:
   (a) überzogene Retardpartikel mit einem Magnesium-haltigen Kern und einem LipidÜberzug, wobei der Kern eine Magnesiumverbindung enthält oder aus dieser besteht, und wobei die Retardpartikel das enthaltene Magnesium in retardierter Weise freisetzen;
   (b) nicht-retardierte Magnesium-haltige Partikel, wobei die Partikel eine Magnesiumverbindung enthalten oder aus dieser bestehen, und wobei die Partikel das enthaltene Magnesium in nicht-retardierter Weise freisetzen;
   (c) einen oder mehrere wasserlösliche Hilfsstoffe ausgewählt aus der Gruppe der Zucker, Zuckeralkohole und Oligosaccharide; und
   (d) optional einen oder mehrere weitere Hilfsstoffe;
      dadurch gekennzeichnet, dass mindestens die Magnesium-haltigen Kerne der Retardpartikel der Komponente (a) eine solche Abriebfestigkeit aufweisen, dass nach einer Vibrationssiebung über einen Zeitraum von 60 Minuten:
      - der Feinanteil an Partikeln mit einem Siebdurchmesser von < 200 µm nach einer Siebzeit von 60 Minuten, t₆₀ₘᵢₙ, um maximal 100 Gew.-% höher ist gegenüber dem initialen Feinanteil dieses Siebdurchmessers nach einer Siebzeit von 5 Minuten, t₅ₘᵢₙ; und/oder
      - der Anteil an Partikeln mit einem Siebdurchmesser von 200 µm ≤ x < 800 µm nach einer Siebzeit von 60 Minuten, t₆₀ₘᵢₙ, um maximal 18 Gew.-% niedriger ist gegenüber dem initialen Anteil dieses Siebdurchmessers nach einer Siebzeit von 5 Minuten, t₅ₘᵢₙ.
2. Zusammensetzung gemäß Ausführungsform 1, wobei mindestens die Magnesium-haltigen Kerne der Retardpartikel der Komponente (a) eine solche Abriebfestigkeit aufweisen, dass nach einer Vibrationssiebung über einen Zeitraum von 60 Minuten:
   - der Feinanteil an Partikeln mit einem Siebdurchmesser von < 200 µm nach einer Siebzeit von 60 Minuten, t₆₀ₘᵢₙ, um maximal 50 Gew.-% höher ist gegenüber dem initialen Feinanteil dieses Siebdurchmessers nach einer Siebzeit von 5 Minuten, t₅ₘᵢₙ; und/oder
   - der Anteil an Partikeln mit einem Siebdurchmesser von 200 µm ≤ x < 800 µm nach einer Siebzeit von 60 Minuten, t₆₀ₘᵢₙ, um maximal 15 Gew.-% niedriger ist gegenüber dem initialen Anteil dieses Siebdurchmessers nach einer Siebzeit von 5 Minuten, t₅ₘᵢₙ.
3. Zusammensetzung gemäß der Ausführungsformen 1 bis 2, wobei mindestens die Magnesium-haltigen Kerne der Retardpartikel der Komponente (a) eine solche Abriebfestigkeit aufweisen, dass nach einer Vibrationssiebung über einen Zeitraum von 60 Minuten:
   - der Feinanteil an Partikeln mit einem Siebdurchmesser von < 200 µm nach einer Siebzeit von 60 Minuten, t₆₀ₘᵢₙ, um maximal 20 Gew.-% höher ist gegenüber dem initialen Feinanteil dieses Siebdurchmessers nach einer Siebzeit von 5 Minuten, t₅ₘᵢₙ; und/oder
   - der Anteil an Partikeln mit einem Siebdurchmesser von 200 µm ≤ x < 800 µm nach einer Siebzeit von 60 Minuten, t₆₀ₘᵢₙ, um maximal 12 Gew.-% niedriger ist gegenüber dem initialen Anteil dieses Siebdurchmessers nach einer Siebzeit von 5 Minuten, t₅ₘᵢₙ.
4. Zusammensetzung gemäß einer der vorhergehenden Ausführungsformen, wobei mindestens die Magnesium-haltigen Kerne der Retardpartikel der Komponente (a) eine solche Abriebfestigkeit aufweisen, dass nach einer Vibrationssiebung über einen Zeitraum von 60 Minuten:
   - der Anteil an Partikeln mit einem Siebdurchmesser von < 300 µm nach einer Siebzeit von 60 Minuten, t₆₀ₘᵢₙ, um maximal 55 Gew.-% höher ist gegenüber dem initialen Anteil dieses Siebdurchmessers nach einer Siebzeit von 5 Minuten, t₅ₘᵢₙ; und/oder
   - der Anteil an Partikeln mit einem Siebdurchmesser von 300 µm ≤ x < 800 µm nach einer Siebzeit von 60 Minuten, t₆₀ₘᵢₙ, um maximal 30 Gew.-% niedriger ist gegenüber dem initialen Anteil dieses Siebdurchmessers nach einer Siebzeit von 5 Minuten, t₅ₘᵢₙ.
5. Zusammensetzung gemäß einer der vorhergehenden Ausführungsformen, wobei mindestens die Magnesium-haltigen Kerne der Retardpartikel der Komponente (a) eine solche Abriebfestigkeit aufweisen, dass nach einer Vibrationssiebung über einen Zeitraum von 60 Minuten:
   - der Anteil an Partikeln mit einem Siebdurchmesser von < 300 µm nach einer Siebzeit von 60 Minuten, t₆₀ₘᵢₙ, um maximal 35 Gew.-% höher ist gegenüber dem initialen Anteil dieses Siebdurchmessers nach einer Siebzeit von 5 Minuten, t₅ₘᵢₙ; und/oder
   - der Anteil an Partikeln mit einem Siebdurchmesser von 300 µm ≤ x < 800 µm nach einer Siebzeit von 60 Minuten, t₆₀ₘᵢₙ, um maximal 27 Gew.-% niedriger ist gegenüber dem initialen Anteil dieses Siebdurchmessers nach einer Siebzeit von 5 Minuten, t₅ₘᵢₙ.
6. Zusammensetzung gemäß einer der vorhergehenden Ausführungsformen, wobei mindestens die Magnesium-haltigen Kerne der Retardpartikel der Komponente (a) eine solche Abriebfestigkeit aufweisen, dass nach einer Vibrationssiebung über einen Zeitraum von 60 Minuten:
   - der Anteil an Partikeln mit einem Siebdurchmesser von < 300 µm nach einer Siebzeit von 60 Minuten, t₆₀ₘᵢₙ, um maximal 15 Gew.-% höher ist gegenüber dem initialen Anteil dieses Siebdurchmessers nach einer Siebzeit von 5 Minuten, t₅ₘᵢₙ; und/oder
   - der Anteil an Partikeln mit einem Siebdurchmesser von 300 µm ≤ x < 800 µm nach einer Siebzeit von 60 Minuten, t₆₀ₘᵢₙ, um maximal 24 Gew.-% niedriger ist gegenüber dem initialen Anteil nach einer Siebzeit von 5 Minuten, t₅ₘᵢₙ.
7. Zusammensetzung gemäß einer der vorhergehenden Ausführungsformen, wobei die Vibrationssiebung mit 100 g der Magnesium-haltigen Kerne und je 1 g Siebhilfe pro Siebboden sowie bei einer Vibrationsamplitude von 80/min durchgeführt wird.
8. Zusammensetzung gemäß Ausführungsform 7, wobei die Siebhilfe in Form abriebfester Silicagel-Kugeln vorliegt, welche einen Durchmesser von 2-5 mm und eine Schüttdichte von ungefähr 750 kg/m³ aufweisen.
9. Zusammensetzung gemäß einer der vorhergehenden Ausführungsformen, wobei die Magnesium-haltigen Kerne der Retardpartikel mittels Trockenkompaktierung hergestellt werden.
10. Zusammensetzung gemäß einer der vorhergehenden Ausführungsformen, wobei die Magnesium-haltigen Kerne der Retardpartikel eine mediane Teilchengröße (D50), gemessen mittels dynamischer Bildanalyse (z. Bsp. mittels Camsizer^{®} XT, Retsch Technology GmbH, Haan, Deutschland), zwischen 150 µm und 300 µm aufweisen, bevorzugt zwischen 165 µm und 285 µm, weiter bevorzugt zwischen 180 µm und 270 µm.
11. Zusammensetzung gemäß einer der vorhergehenden Ausführungsformen, wobei die Magnesium-haltigen Kerne der Retardpartikel eine unimodale Partikelgrößenverteilung aufweisen.
12. Zusammensetzung gemäß einer der vorhergehenden Ausführungsformen, wobei die Magnesium-haltigen Kerne der Retardpartikel eine so enge Partikelgrößenverteilung aufweisen, dass der Quotient (D90-D10) / D50 unter 1,60 liegt, bevorzugt unter 1,50, und weiterhin bevorzugt unter 1,40.
13. Zusammensetzung gemäß einer der vorhergehenden Ausführungsformen, wobei mindestens die Magnesiumverbindung in den Retardpartikeln der Komponente (a) in ihrer wasserfreien Form einen Magnesiumgehalt von 15 Gew.-% oder mehr aufweist, bevorzugt 25 Gew.-% oder mehr, weiterhin bevorzugt 50 Gew.-% oder mehr.
14. Zusammensetzung gemäß einer der vorhergehenden Ausführungsformen, wobei die Magnesiumverbindung in den Retardpartikeln ausgewählt ist aus Magnesiumoxid (MgO), Magnesiumcarbonat (MgCO₃), Magnesiumchlorid (MgCl₂),Magnesiumhydrogenphosphat (MgHPO₄) und/oder Magnesiumacetat (Mg(CH₃COO)₂).
15. Zusammensetzung gemäß einer der vorhergehenden Ausführungsformen, wobei der Kern der Retardpartikel optional einen oder mehrere Hilfsstoffe enthält.
16. Zusammensetzung gemäß einer der vorhergehenden Ausführungsformen, wobei die Magnesiumverbindung im Kern der Retardpartikel Magnesiumoxid (MgO) ist.
17. Zusammensetzung gemäß einer der vorhergehenden Ausführungsformen, wobei der Kern in den Retardpartikeln im Wesentlichen aus Magnesiumoxid (MgO) besteht.
18. Zusammensetzung gemäß einer der vorhergehenden Ausführungsformen, wobei der Überzug der Retardpartikel ein Lipid mit einem Schmelzpunkt von mindestens 50 °C enthält, oder im Wesentlichen hieraus besteht; bevorzugt ein Lipid mit einem Schmelzpunkt von mindestens 60 °C.
19. Zusammensetzung gemäß einer der vorhergehenden Ausführungsformen, wobei es sich bei den Lipiden im Überzug der Retardpartikel um mindestens ein Triglycerid handelt.
20. Zusammensetzung gemäß einer der vorhergehenden Ausführungsformen, wobei der Überzug der Retardpartikel mindestens 70 Gew.-%, oder mindestens 80 Gew.-%, oder mindestens 90 Gew.-% eines Triglycerid enthält.
21. Zusammensetzung gemäß einer der Ausführungsformen 19 oder 20, wobei das Triglycerid natürliche Fettsäuren enthält.
22. Zusammensetzung gemäß einer der vorhergehenden Ausführungsformen, wobei der Überzug der Retardpartikel Glyceroltripalmitat und/oder Glyceroltristearat enthält, oder im Wesentlichen hieraus besteht.
23. Zusammensetzung gemäß einer der vorhergehenden Ausführungsformen, wobei der Überzug der Retardpartikel mindestens 70 Gew.-%, oder mindestens 80 Gew.-%, oder mindestens 90 Gew.-% Glyceroltripalmitat und/oder Glyceroltristearat enthält.
24. Zusammensetzung gemäß einer der vorhergehenden Ausführungsformen, wobei der Überzug der Retardpartikel im Wesentlichen aus Glyceroltripalmitat und/oder Glyceroltristearat besteht.
25. Zusammensetzung gemäß einer der vorhergehenden Ausführungsformen, wobei der Überzug der Retardpartikel im Wesentlichen aus Glyceroltristearat besteht.
26. Zusammensetzung gemäß einer der vorhergehenden Ausführungsformen, wobei das Gewichtsverhältnis zwischen Kern und Überzug in den Retardpartikeln zwischen 60:40 und 95:5 liegt, oder zwischen 70:30 und 90:10 liegt.
27. Zusammensetzung gemäß einer der vorhergehenden Ausführungsformen, wobei die Stärke des Überzugs in den Retardpartikeln so gewählt ist, dass die Retardpartikel innerhalb von 4 Stunden, bevorzugt innerhalb von 5 Stunden, weiter bevorzugt innerhalb von 6 Stunden, nicht mehr als 80 % des in ihnen enthaltenen Magnesiums freisetzen, gemessen in einer Freisetzungs-Apparatur mit Blattrührer (USP Dissolution Apparatus 2) in 900 mL 0.1 N Salzsäure bei 37 °C und einer Rührgeschwindigkeit von 75 UPM.
28. Zusammensetzung gemäß einer der vorhergehenden Ausführungsformen, wobei die Magnesiumverbindung in den nicht-retardierten Partikeln der Komponente (b) ausgewählt ist aus Magnesiumoxid (MgO), Magnesiumcarbonat (MgCO₃), Magnesiumacetat (Mg(CH₃COO)₂), Magnesiumhydrogenphosphat (MgHPO₄) Magnesiumhydrogencitrat, Trimagnesiumdicitrat (C₁₂H₁₀Mg₃O₁₄), Magnesiumpidolat (C₁₀H₁₂MgN₂O₆), Magnesium N-Acetyltaurinat (C₈H₁₆MgN₂O₈S₂) und/oder Magnesium-bis(hydrogen-L-glutamat), sowie deren Hydraten.
29. Zusammensetzung gemäß einer der vorhergehenden Ausführungsformen, wobei die Magnesiumverbindung in den nicht-retardierten Partikeln der Komponente (b) ausgewählt ist aus Magnesiumhydrogencitrat und/oder Trimagnesiumdicitrat.
30. Zusammensetzung gemäß einer der vorhergehenden Ausführungsformen, wobei die nicht-retardierten Partikel der Komponente (b) im Wesentlichen aus Magnesium-hydrogencitrat und/oder Trimagnesiumdicitrat bestehen.
31. Zusammensetzung gemäß einer der vorhergehenden Ausführungsformen, wobei die Retardpartikel der Komponente (a) eine andere Magnesiumverbindung enthalten als die nicht-retardierten Partikel der Komponente (b).
32. Zusammensetzung gemäß Ausführungsform 31, wobei der Kern der Retardpartikel Magnesiumoxid (MgO) enthält, und die nicht-retardierten Partikel der Komponente (b) Magnesiumhydrogencitrat und/oder Trimagnesiumdicitrat enthalten.
33. Zusammensetzung gemäß Ausführungsform 32, wobei der Kern der Retardpartikel im Wesentlichen aus Magnesiumoxid (MgO) besteht, und die nicht-retardierten Partikel der Komponente (b) im Wesentlichen aus Magnesiumhydrogen-citrat und/oder Trimagnesiumdicitrat bestehen.
34. Zusammensetzung gemäß einer der vorhergehenden Ausführungsformen, wobei die Retardpartikel der Komponente (a), und optional die nicht-retardierten Partikel der Komponente (b), eine mediane Teilchengröße (D50), gemessen mittels dynamischer Bildanalyse (z. Bsp. mittels Camsizer^{®} XT, Retsch Technology GmbH, Haan, Deutschland), von ≤ 500 µm aufweisen, bevorzugt ≤ 450 µm, weiter bevorzugt ≤ 400 µm.
35. Zusammensetzung gemäß Ausführungsform 34, wobei die Retardpartikel der Komponente (a), und optional die nicht-retardierten Partikel der Komponente (b), eine mediane Teilchengröße (D50), gemessen mittels dynamischer Bildanalyse, zwischen 100 µm und 500 µm aufweisen, bevorzugt zwischen 150 µm und 450 µm, weiterhin bevorzugt zwischen 200 µm und 400 µm.
36. Zusammensetzung gemäß einer der vorhergehenden Ausführungsformen, wobei die Retardpartikel der Komponente (a) zwischen 15 und 85 Gew.-%, oder zwischen 25 und 75 Gew.-%, oder zwischen 30 und 70 Gew.-%, oder zwischen 30 und 60 Gew.-% der Zusammensetzung ausmachen.
37. Zusammensetzung gemäß einer der vorhergehenden Ausführungsformen, wobei diese mindestens 20 Gew.-%, bevorzugt mindestens 23 Gew.-%, weiterhin bevorzugt mindestens 25 Gew.-% der Hilfsstoffe gemäß Komponente (c) enthält.
38. Zusammensetzung gemäß einer der vorhergehenden Ausführungsformen, wobei die Hilfsstoffe der Komponente (c) mindestens 60 Gew.-%, bevorzugt mindestens 75 Gew.-%, weiterhin bevorzugt mindestens 85 Gew.-%, eines in Wasser löslichen Zuckers oder Zuckeralkohols enthalten, oder optional hieraus bestehen.
39. Zusammensetzung gemäß Ausführungsform 38, wobei der in Wasser lösliche Zucker oder Zuckeralkohol ausgewählt ist aus Saccharose, Sorbitol, Xylitol, Erythritol, Maltitol, Isomaltitol, Mannitol, und Mischungen derselben.
40. Zusammensetzung gemäß einer der vorhergehenden Ausführungsformen, wobei die Zusammensetzung als weitere Hilfsstoffe gemäß Komponente (d) Zitronensäure, Mononatriumcitrat und/oder Dinatriumcitrat enthalten, insbesondere Zitronensäure in Kombination mit Mononatriumcitrat.
41. Zusammensetzung gemäß einer der vorhergehenden Ausführungsformen, wobei die Mengen der Retardpartikel der Komponente (a) und der nicht-retardierten Partikel der Komponente (b) so gewählt sind, dass innerhalb von 4 Stunden, bevorzugt innerhalb von 5 Stunden, weiter bevorzugt innerhalb von 6 Stunden, nicht mehr als 80 % des in der Zusammensetzung enthaltenen Magnesiums freisetzen, gemessen in einer Freisetzungs-Apparatur mit Blattrührer (USP Dissolution Apparatus 2) in 900 mL 0.1 N Salzsäure bei 37 °C und einer Rührgeschwindigkeit von 75 UPM.
42. Zusammensetzung gemäß einer der vorhergehenden Ausführungsformen, wobei mindestens 60 Gew.-%, oder mindestens 75 Gew.-%, oder mindestens 85 Gew.-%, des gesamten in der Zusammensetzung enthaltenen Magnesiums in den Retardpartikeln der Komponente (a) enthalten ist.
43. Zusammensetzung gemäß einer der vorhergehenden Ausführungsformen, wobei die Zusammensetzung mindestens 5 Gew.-%, oder mindestens 10 Gew.-%, oder mindestens 12 Gew.-% des gesamten in der Zusammensetzung enthaltenen Magnesiums in nicht-retardierter Form enthält.
44. Zusammensetzung gemäß einer der vorhergehenden Ausführungsformen, wobei die Komponenten gemäß (b), (c) und/oder (d) optional in granulierter Form vorliegen.
45. Zusammensetzung gemäß einer der vorhergehenden Ausführungsformen, wobei die Hilfsstoffe der Komponenten (c) und (d) nicht mehr als 5 Gew.-% von in Wasser schwerlöslichen Hilfsstoffen enthalten.
46. Zusammensetzung gemäß einer der vorhergehenden Ausführungsformen, wobei Einzeldosen der Zusammensetzung in Stickpacks, Sachets, Glasampullen oder Plastikampullen verpackt sind.
47. Verfahren zur Herstellung einer festen pharmazeutischen oder nutrazeutischen Zusammensetzung gemäß der Ausführungsformen 1 bis 46, enthaltend die folgenden Schritte:
   (i) Bereitstellen eines Magnesium-haltigen Kerns, wobei der Kern eine Magnesiumverbindung enthält oder aus dieser besteht;
   (ii) Bereitstellen eines geschmolzenen Überzugsmaterials enthaltend ein Lipid;
   (iii) Fluidisieren der Magnesium-haltigen Kerne;
   (iv) Besprühen der fluidisierten Magnesium-haltigen Kerne mit dem geschmolzenen Überzugsmaterial;
   (v) Abkühlen der überzogenen Magnesium-haltigen Kerne, so dass das Lipid erstarrt und Retardpartikel gemäß Komponente (a) erhalten werden, welche das enthaltene Magnesium in retardierter Weise freisetzen; und
   (vi) Mischen der so als Komponente (a) erhaltenen Retardpartikel mit den folgenden weiteren Komponenten:
      (b) nicht-retardierte Partikel mit einem zweiten Magnesium-haltigen Kern, wobei der Partikel eine Magnesiumverbindung enthält oder aus dieser besteht, und wobei die Partikel das enthaltene Magnesium in nicht-retardierter Weise freisetzen;
      (c) einen oder mehrere wasserlösliche Hilfsstoffe ausgewählt aus der Gruppe der Zucker, Zuckeralkohole und Oligosaccharide; und
      (d) optional weiteren Hilfsstoffen;
         dadurch gekennzeichnet, dass mindestens die Magnesium-haltigen Kerne der Retardpartikel der Komponente (a) eine solche Abriebfestigkeit aufweisen, dass nach einer Vibrationssiebung über einen Zeitraum von 60 Minuten:
         - der Feinanteil an Partikeln mit einem Siebdurchmesser von < 200 µm nach einer Siebzeit von 60 Minuten, t₆₀ₘᵢₙ, um maximal 100 Gew.-% höher ist gegenüber dem initialen Feinanteil dieses Siebdurchmessers nach einer Siebzeit von 5 Minuten, t₅ₘᵢₙ; und/oder
         - der Anteil an Partikeln mit einem Siebdurchmesser von 200 µm ≤ x < 800 µm nach einer Siebzeit von 60 Minuten, t₆₀ₘᵢₙ, um maximal 18 Gew.-% niedriger ist gegenüber dem initialen Anteil dieses Siebdurchmessers nach einer Siebzeit von 5 Minuten, t₅ₘᵢₙ.
48. Verfahren gemäß Ausführungsform 47, wobei mindestens die im Schritt (i) bereitgestellten Magnesium-haltigen Kerne der Retardpartikel der Komponente (a) eine solche Abriebfestigkeit aufweisen, dass nach einer Vibrationssiebung über einen Zeitraum von 60 Minuten:
   - der Feinanteil an Partikeln mit einem Siebdurchmesser von < 200 µm nach einer Siebzeit von 60 Minuten, t₆₀ₘᵢₙ, um maximal 50 Gew.-% höher ist gegenüber dem initialen Feinanteil dieses Siebdurchmessers nach einer Siebzeit von 5 Minuten, t₅ₘᵢₙ; und/oder
   - der Anteil an Partikeln mit einem Siebdurchmesser von 200 µm ≤ x < 800 µm nach einer Siebzeit von 60 Minuten, t₆₀ₘᵢₙ, um maximal 15 Gew.-% niedriger ist
   gegenüber dem initialen Anteil dieses Siebdurchmessers nach einer Siebzeit von 5 Minuten, t₅ₘᵢₙ.
49. Verfahren gemäß der Ausführungsformen 47 bis 48, wobei mindestens die im Schritt (i) bereitgestellten Magnesium-haltigen Kerne der Retardpartikel der Komponente (a) eine solche Abriebfestigkeit aufweisen, dass nach einer Vibrationssiebung über einen Zeitraum von 60 Minuten:
   - der Feinanteil an Partikeln mit einem Siebdurchmesser von < 200 µm nach einer Siebzeit von 60 Minuten, t₆₀ₘᵢₙ, um maximal 20 Gew.-% höher ist gegenüber dem initialen Feinanteil dieses Siebdurchmessers nach einer Siebzeit von 5 Minuten, t₅ₘᵢₙ; und/oder
   - der Anteil an Partikeln mit einem Siebdurchmesser von 200 µm ≤ x < 800 µm nach einer Siebzeit von 60 Minuten, t₆₀ₘᵢₙ, um maximal 12 Gew.-% niedriger ist gegenüber dem initialen Anteil dieses Siebdurchmessers nach einer Siebzeit von 5 Minuten, t₅ₘᵢₙ.
50. Verfahren gemäß einer der vorhergehenden Ausführungsformen, wobei mindestens die im Schritt (i) bereitgestellten Magnesium-haltigen Kerne der Retardpartikel der Komponente (a) eine solche Abriebfestigkeit aufweisen, dass nach einer Vibrationssiebung über einen Zeitraum von 60 Minuten:
   - der Anteil an Partikeln mit einem Siebdurchmesser von < 300 µm nach einer Siebzeit von 60 Minuten, t₆₀ₘᵢₙ, um maximal 55 Gew.-% höher ist gegenüber dem initialen Anteil dieses Siebdurchmessers nach einer Siebzeit von 5 Minuten, t₅ₘᵢₙ; und/oder
   - der Anteil an Partikeln mit einem Siebdurchmesser von 300 µm ≤ x < 800 µm nach einer Siebzeit von 60 Minuten, t₆₀ₘᵢₙ, um maximal 30 Gew.-% niedriger ist gegenüber dem initialen Anteil dieses Siebdurchmessers nach einer Siebzeit von 5 Minuten, t₅ₘᵢₙ.
51. Verfahren gemäß einer der vorhergehenden Ausführungsformen, wobei mindestens die im Schritt (i) bereitgestellten Magnesium-haltigen Kerne der Retardpartikel der Komponente (a) eine solche Abriebfestigkeit aufweisen, dass nach einer Vibrationssiebung über einen Zeitraum von 60 Minuten:
   - der Anteil an Partikeln mit einem Siebdurchmesser von < 300 µm nach einer Siebzeit von 60 Minuten, t₆₀ₘᵢₙ, um maximal 35 Gew.-% höher ist gegenüber dem initialen Anteil dieses Siebdurchmessers nach einer Siebzeit von 5 Minuten, t₅ₘᵢₙ;
      und/oder
   - der Anteil an Partikeln mit einem Siebdurchmesser von 300 µm ≤ x < 800 µm nach einer Siebzeit von 60 Minuten, t₆₀ₘᵢₙ, um maximal 27 Gew.-% niedriger ist gegenüber dem initialen Anteil dieses Siebdurchmessers nach einer Siebzeit von 5 Minuten, t₅ₘᵢₙ.
52. Verfahren gemäß einer der vorhergehenden Ausführungsformen, wobei mindestens die im Schritt (i) bereitgestellten Magnesium-haltigen Kerne der Retardpartikel der Komponente (a) eine solche Abriebfestigkeit aufweisen, dass nach einer Vibrationssiebung über einen Zeitraum von 60 Minuten:
   - der Anteil an Partikeln mit einem Siebdurchmesser von < 300 µm nach einer Siebzeit von 60 Minuten, t₆₀ₘᵢₙ, um maximal 15 Gew.-% höher ist gegenüber dem initialen Anteil dieses Siebdurchmessers nach einer Siebzeit von 5 Minuten, t₅ₘᵢₙ; und/oder
   - der Anteil an Partikeln mit einem Siebdurchmesser von 300 µm ≤ x < 800 µm nach einer Siebzeit von 60 Minuten, t₆₀ₘᵢₙ, um maximal 24 Gew.-% niedriger ist gegenüber dem initialen Anteil nach einer Siebzeit von 5 Minuten, t₅ₘᵢₙ.
53. Verfahren gemäß einer der vorhergehenden Ausführungsformen, wobei die Vibrationssiebung mit 100 g der Magnesium-haltigen Kerne und je 1 g Siebhilfe pro Siebboden sowie bei einer Vibrationsamplitude von 80/min durchgeführt wird.
54. Zusammensetzung gemäß Ausführungsform 53, wobei die Siebhilfe in Form abriebfester Silicagel-Kugeln vorliegt, welche einen Durchmesser von 2-5 mm und eine Schüttdichte von ungefähr 750 kg/m³ aufweisen.
55. Verfahren gemäß einer der vorhergehenden Ausführungsformen, wobei die im Schritt (i) bereitgestellten Magnesium-haltigen Kerne der Retardpartikel mittels Trockenkompaktierung hergestellt werden.
56. Verfahren gemäß einer der vorhergehenden Ausführungsformen, wobei die im Schritt (i) bereitgestellten Magnesium-haltigen Kerne der Retardpartikel eine mediane Teilchengröße (D50), gemessen mittels dynamischer Bildanalyse (z. Bsp. mittels Camsizer^{®} XT, Retsch Technology GmbH, Haan, Deutschland), zwischen 150 µm und 300 µm aufweisen, bevorzugt zwischen 165 µm und 285 µm, weiter bevorzugt zwischen 180 µm und 270 µm.
57. Verfahren gemäß einer der vorhergehenden Ausführungsformen, wobei die im Schritt (i) bereitgestellten Magnesium-haltigen Kerne der Retardpartikel eine unimodale Partikelgrößenverteilung aufweisen.
58. Verfahren gemäß einer der vorhergehenden Ausführungsformen, wobei die im Schritt (i) bereitgestellten Magnesium-haltigen Kerne der Retardpartikel eine so enge Partikelgrößenverteilung aufweisen, dass der Quotient (D90-D10) / D50 unter 1,60 liegt, bevorzugt unter 1,50, und weiterhin bevorzugt unter 1,40.
59. Verfahren gemäß einer der vorhergehenden Ausführungsformen, wobei die im Schritt (i) bereitgestellten Retardpartikel der Komponente (a) eine andere Magnesiumverbindung enthalten als die im Schritt (vi) zugemischten nicht-retardierten Partikel der Komponente (b).
60. Verfahren gemäß einer der vorhergehenden Ausführungsformen, wobei mindestens die Magnesiumverbindung in den Retardpartikeln der Komponente (a) in ihrer wasserfreien Form einen Magnesiumgehalt von 15 Gew.-% oder mehr aufweist, bevorzugt 25 Gew.-% oder mehr, weiterhin bevorzugt 50 Gew.-% oder mehr.
61. Verfahren gemäß einer der vorhergehenden Ausführungsformen, wobei die Magnesiumverbindung in den Retardpartikeln ausgewählt ist aus Magnesiumoxid (MgO), Magnesiumcarbonat (MgCO₃),Magnesiumchlorid (MgCl₂), Magnesiumhydrogenphosphat (MgHPO₄) und/oder Magnesiumacetat (Mg(CH₃COO)₂).
62. Verfahren gemäß einer der vorhergehenden Ausführungsformen, wobei der Kern der Retardpartikel optional einen oder mehrere Hilfsstoffe enthält.
63. Verfahren gemäß einer der vorhergehenden Ausführungsformen, wobei die Magnesiumverbindung im Kern der Retardpartikel Magnesiumoxid (MgO) ist.
64. Verfahren gemäß einer der vorhergehenden Ausführungsformen, wobei der Kern in den Retardpartikeln im Wesentlichen aus Magnesiumoxid (MgO) besteht.
65. Verfahren gemäß einer der vorhergehenden Ausführungsformen, wobei das im Schritt (ii) bereitgestellte Überzugsmaterial der Retardpartikel ein Lipid mit einem Schmelzpunkt von mindestens 50 °C enthält, oder im Wesentlichen hieraus besteht; bevorzugt ein Lipid mit einem Schmelzpunkt von mindestens 60 °C.
66. Verfahren gemäß einer der vorhergehenden Ausführungsformen, wobei es sich bei den Lipiden im Überzugsmaterial der Retardpartikel um mindestens ein Triglycerid handelt.
67. Verfahren gemäß einer der vorhergehenden Ausführungsformen, wobei das im Schritt (ii) bereitgestellte Überzugsmaterial der Retardpartikel mindestens 70 Gew.-%, oder mindestens 80 Gew.-%, oder mindestens 90 Gew.-% eines Triglycerids enthält.
68. Verfahren gemäß der Ausführungsformen 66 oder 67, wobei das Triglycerid natürliche Fettsäuren enthält.
69. Verfahren gemäß einer der vorhergehenden Ausführungsformen, wobei das im Schritt (ii) bereitgestellte Überzugsmaterial der Retardpartikel Glyceroltripalmitat und/oder Glyceroltristearat enthält, oder im Wesentlichen hieraus besteht.
70. Verfahren gemäß einer der vorhergehenden Ausführungsformen, wobei das im Schritt (ii) bereitgestellte Überzugsmaterial der Retardpartikel mindestens 70 Gew.-%, oder mindestens 80 Gew.-%, oder mindestens 90 Gew.-% Glyceroltripalmitat und/oder Glyceroltristearat enthält.
71. Verfahren gemäß einer der vorhergehenden Ausführungsformen, wobei das im Schritt (ii) bereitgestellte Überzugsmaterial der Retardpartikel im Wesentlichen aus Glyceroltripalmitat und/oder Glyceroltristearat besteht.
72. Verfahren gemäß einer der vorhergehenden Ausführungsformen, wobei das im Schritt (ii) bereitgestellte Überzugsmaterial der Retardpartikel im Wesentlichen aus Glyceroltristearat besteht.
73. Verfahren gemäß einer der vorhergehenden Ausführungsformen, wobei im Schritt (iv) das Gewichtsverhältnis zwischen den fluidisierten Magnesium-haltigen Kernen und dem geschmolzenen Überzugsmaterial zwischen 60:40 und 95:5 liegt, oder zwischen 70:30 und 90:10 liegt.
74. Verfahren gemäß einer der vorhergehenden Ausführungsformen, wobei die Stärke des in den Schritten (iv) und (v) aufgetragenen Überzugs so gewählt ist, dass die Retardpartikel innerhalb von 4 Stunden, bevorzugt innerhalb von 5 Stunden, weiter bevorzugt innerhalb von 6 Stunden, nicht mehr als 80 % des in ihnen enthaltenen Magnesiums freisetzen, gemessen in einer Freisetzungs-Apparatur mit Blattrührer (USP Dissolution Apparatus 2) in 900 mL 0.1 N Salzsäure bei 37 °C und einer Rührgeschwindigkeit von 75 UPM.
75. Verfahren gemäß einer der vorhergehenden Ausführungsformen, wobei die Magnesiumverbindung in den im Schritt (vi) zugemischten, nicht-retardierten Partikeln der Komponente (b) ausgewählt ist aus Magnesiumoxid (MgO), Magnesiumcarbonat (MgCO₃),Magnesiumacetat (Mg(CH₃COO)₂), Magnesiumhydrogenphosphat (MgHPO₄), Magnesiumhydrogencitrat, Trimagnesiumdicitrat (C₁₂H₁₀Mg₃O₁₄), Magnesiumpidolat (C₁₀H₁₂MgN₂O₆), Magnesium N-Acetyltaurinat (C₈H₁₆MgN₂O₈S₂) und/oder Magnesium-bis(hydrogen-L-glutamat), sowie deren Hydraten.
76. Verfahren gemäß einer der vorhergehenden Ausführungsformen, wobei die Magnesiumverbindung in den im Schritt (vi) zugemischten, nicht-retardierten Partikeln der Komponente (b) ausgewählt ist aus Magnesiumhydrogencitrat und/oder Trimagnesiumdicitrat.
77. Verfahren gemäß einer der vorhergehenden Ausführungsformen, wobei die im Schritt (vi) zugemischten, nicht-retardierten Partikel der Komponente (b) im Wesentlichen aus Magnesiumhydrogencitrat und/oder Trimagnesiumdicitrat bestehen.
78. Verfahren gemäß einer der vorhergehenden Ausführungsformen, wobei die Retardpartikel der Komponente (a) eine andere Magnesiumverbindung enthalten als die im Schritt (vi) zugemischten nicht-retardierten Partikel der Komponente (b).
79. Zusammensetzung gemäß Ausführungsform 78, wobei der Kern der Retardpartikel Magnesiumoxid (MgO) enthält, und die im Schritt (vi) zugemischten nicht-retardierten Partikel der Komponente (b) Magnesiumhydrogencitrat und/oder Trimagnesiumdicitrat enthalten.
80. Zusammensetzung gemäß Ausführungsform 79, wobei der Kern der Retardpartikel im Wesentlichen aus Magnesiumoxid (MgO) besteht, und die im Schritt (vi) zugemischten nicht-retardierten Partikel der Komponente (b) im Wesentlichen aus Magnesiumhydrogen-citrat und/oder Trimagnesiumdicitrat bestehen.
81. Verfahren gemäß einer der vorhergehenden Ausführungsformen, wobei die Retardpartikel der Komponente (a), und optional die im Schritt (vi) zugemischten, nicht-retardierten Partikel der Komponente (b), eine mediane Teilchengröße (D50), gemessen mittels dynamischer Bildanalyse (z. Bsp. mittels Camsizer^{®} XT, Retsch Technology GmbH, Haan, Deutschland), von ≤ 500 µm auf, bevorzugt ≤ 450 µm, weiter bevorzugt ≤ 400 µm
82. Verfahren gemäß Ausführungsform 81, wobei die Retardpartikel der Komponente (a), und optional die nicht-retardierten Partikel der Komponente (b), eine mediane Teilchengröße (D50), gemessen mittels dynamischer Bildanalyse, zwischen 100 µm und 500 µm aufweisen, bevorzugt zwischen 150 µm und 450 µm, weiterhin bevorzugt zwischen 200 µm und 400 µm.
83. Verfahren gemäß einer der vorhergehenden Ausführungsformen, wobei im Schritt (vi) die Retardpartikel der Komponente (a) zwischen 15 und 85 Gew.-%, oder zwischen 25 und 75 Gew.-%oder zwischen 30 und 70 Gew.-%, oder zwischen 30 und 60 Gew.-% der Zusammensetzung ausmachen.
84. Verfahren gemäß einer der vorhergehenden Ausführungsformen, wobei im Schritt (vi) die Hilfsstoffe der Komponente (c) mindestens 20 Gew.-%, bevorzugt mindestens 23 Gew.-%, weiterhin bevorzugt mindestens 25 Gew.-% der Zusammensetzung ausmachen.
85. Verfahren gemäß einer der vorhergehenden Ausführungsformen, wobei im Schritt (vi) die Hilfsstoffe der Komponente (c) mindestens 60 Gew.-%, bevorzugt mindestens 75 Gew.-%, weiterhin bevorzugt mindestens 85 Gew.-%, eines in Wasser löslichen Zuckers oder Zuckeralkohols enthalten, oder optional hieraus bestehen.
86. Verfahren gemäß Ausführungsform 85, wobei der in Wasser lösliche Zucker oder Zuckeralkohol ausgewählt ist aus Saccharose, Sorbitol, Xylitol, Erythritol, Maltitol, Isomaltitol, Mannitol, und Mischungen derselben.
87. Verfahren gemäß einer der vorhergehenden Ausführungsformen, wobei im Schritt (vi) die Zusammensetzung als weitere Hilfsstoffe gemäß Komponente (d) Zitronensäure, Mononatriumcitrat und/oder Dinatriumcitrat enthalten, insbesondere Zitronensäure in Kombination mit Mononatriumcitrat.
88. Verfahren gemäß einer der vorhergehenden Ausführungsformen, wobei im Schritt (vi) die Mengen der Retardpartikel der Komponente (a) und der nicht-retardierten Partikel der Komponente (b) so gewählt sind, dass innerhalb von 4 Stunden, bevorzugt innerhalb von 5 Stunden, weiter bevorzugt innerhalb von 6 Stunden, nicht mehr als 80 % des in der Zusammensetzung enthaltenen Magnesiums freisetzen, gemessen in einer Freisetzungs-Apparatur mit Blattrührer (USP Dissolution Apparatus 2) in 900 mL 0.1 N Salzsäure bei 37 °C und einer Rührgeschwindigkeit von 75 UPM.
89. Verfahren gemäß einer der vorhergehenden Ausführungsformen, wobei mindestens 60 Gew.-%, oder mindestens 75 Gew.-%, oder mindestens 85 Gew.-%, des gesamten in der Zusammensetzung enthaltenen Magnesiums in den Retardpartikeln der Komponente (a) enthalten ist.
90. Verfahren gemäß einer der vorhergehenden Ausführungsformen, wobei die Zusammensetzung mindestens 5 Gew.-%, oder mindestens 10 Gew.-%, oder mindestens 12 Gew.-% des gesamten in der Zusammensetzung enthaltenen Magnesiums in nicht-retardierter Form enthält.
91. Verfahren gemäß einer der vorhergehenden Ausführungsformen, wobei die im Schritt (vi) zugemischten Komponenten gemäß (b), (c) und/oder (d) optional in granulierter Form vorliegen.
92. Verfahren gemäß einer der vorhergehenden Ausführungsformen, wobei die Hilfsstoffe der im Schritt (vi) zugemischten Komponenten (c) und (d) nicht mehr als 5 Gew.-% von in Wasser schwerlöslichen Hilfsstoffen enthalten.
93. Verfahren gemäß einer der vorhergehenden Ausführungsformen, wobei die Prozesslufttemperatur in den Schritten (iii) und/oder (iv) zwischen 20 und 60 °C, oder zwischen 20 und 50 °C, oder zwischen 20 und 45 °C liegt; bspw. bei ca. 25 °C oder 35 °C.
94. Verfahren gemäß einer der vorhergehenden Ausführungsformen, wobei das in Schritt (ii) bereitgestellte geschmolzene Überzugsmaterial im Schritt (iv) bei einer Schmelztemperatur zwischen 70 und 120 °C, oder zwischen 75 und 110 °C, oder zwischen 80 und 100 °C versprüht wird; bspw. bei ca. 90 bis 100 °C.
95. Verfahren gemäß einer der vorhergehenden Ausführungsformen, wobei im Schritt (iv) eine Sprühlufttemperatur zwischen 70 und 130 °C, oder zwischen 75 und 125 °C, oder zwischen 80 und 120 °C verwendet wird; bspw. ca. 100 °C oder 120 °C.
96. Verfahren gemäß einer der vorhergehenden Ausführungsformen, wobei der Sprühdruck im Schritt (iv) zwischen 0,5 und 1,5 bar, oder zwischen 0,6 und 1,3 bar, oder zwischen 0,7 und 1,2 bar liegt; bspw. bei 0,8 bis 1,2 bar, 0,8 bis 0,9 bar oder 1,0 bis 1,2 bar.
97. Verfahren gemäß einer der vorhergehenden Ausführungsformen, wobei die Sprühmenge des geschmolzenen Überzugsmaterials pro Kilogramm zu überziehendem Kern-Material im Schritt (iv) zwischen 2,0 und 10,0 g/kg/min, oder zwischen 2,5 und 9,0 g/kg/min, oder zwischen 3,0 und 8,0 g/kg/min liegt;
   bspw. bei ca. 3,3 g/kg/min.
98. Verfahren gemäß einer der vorhergehenden Ausführungsformen, wobei das Verfahren, insbesondere die Überzugsschritte (iii) bis (v) in einem Wirbelschichtgerät im Labor-Maßstab (z. Bsp. in einem Ventilus^{®}V-2.5) durchgeführt wird, und wobei die Sprühmenge des geschmolzenen Überzugsmaterials im Schritt (iv) zwischen 5,0 und 8,0 g/min, oder zwischen 5,5 und 7,5 g/min, oder zwischen 6,0 und 7,0 g/min liegt; bspw. bei ca. 6,5 g/min.
99. Verfahren gemäß einer der vorhergehenden Ausführungsformen, wobei in einem weiteren Schritt, im Anschluss an Schritt (vi), Einzeldosen der Zusammensetzung in Stickpacks, Sachets, Glasampullen oder Plastikampullen verpackt werden.
100. Feste pharmazeutische oder nutrazeutische Zusammensetzung in Form eines oralen Direktgranulats mit mindestens dualer Wirkstofffreisetzung, hergestellt durch das Verfahren gemäß der Ausführungsformen 47 bis 99.

### BEISPIELE

### Beispiel 1: Vergleich der Eigenschaften verschiedener MgO-Kerne

Granulierte Magnesiumoxid (MgO) Partikel verschiedener Anbieter wurde unter anderem auf ihre Partikelgröße und ihre Abriebfestigkeit hin untersucht, um zu prüfen, welche Ware sich zur Verwendung als Kern der Retardpartikel in einem Hotmelt-Coating Prozess eignet; darunter u.a. trockenkompaktierte Rohwaren wie das ,Heavy Magnesium Oxide EP' von Kyowa Chemical Industry Co., Ltd (Japan; nachstehend als ,MgO 1' bezeichnet) und MagGran^{®} MO der Magnesia GmbH (Deutschland; nachstehend ,MgO 2'). Um den Abrieb der MgO-Kerne während des Hot-Melt Coatings in der Wirbelschicht zu simulieren, wurden diese für insgesamt 60 Minuten lang auf einem Siebturm (Retsch AS 200 basic, Retsch GmbH, Haan) mit Siebdurchmesser von 20 cm mechanisch gestresst, wobei zusätzlich eine Siebhilfe in Form von abriebfesten Silicagel-Kugeln verwendet wurde, welche einen Durchmesser von 2-5 mm und eine Schüttdichte von ungefähr 750 kg/m³ aufweisen. Hierbei wurde zunächst eine Probenmenge von ca. 100 g der MgO-Kerne auf das oberste Sieb (800 µm) gegeben und dazu je 1 g Siebhilfe pro Siebboden. Für die Siebung wurde eine Vibrationsamplitude von 80/min verwendet. Alle 5 Minuten wurden die einzelnen Siebe (800 µm, 600 µm, 500 µm, 400 µm, 300 µm, 200 µm, 100 µm und Rückstandsschale) sowie die darauf befindliche Menge an MgO-Kernen gewogen. Die Daten der beiden Siebanalysen sind in Tabellen 1a (MgO1) und 1b (MgO 2) sowie in den Abb. 1a und 1b dargestellt.

**Tabelle 1a: Siebanalyse der MgO 1-Kerne**

| **Sieb [µm]** | **Siebdauer [min]** | | | | | | | | | | | |
|---|---|---|---|---|---|---|---|---|---|---|---|---|
| | **5** | **10** | **15** | **20** | **25** | **30** | **35** | **40** | **45** | **50** | **55** | **60** |
| **800** | 0,0 | 0,0 | 0,0 | 0,0 | 0,0 | 0,0 | 0,0 | 0,0 | 0,0 | 0,0 | 0,0 | 0,0 |
| **600** | 0,0 | 0,0 | 0,0 | 0,0 | 0,0 | 0,0 | 0,0 | 0,0 | 0,0 | 0,0 | 0,0 | 0,0 |
| **500** | 0,1 | 0,0 | 0,0 | 0,0 | 0,0 | 0,0 | 0,0 | 0,0 | 0,0 | 0,0 | 0,0 | 0,1 |
| **400** | 6,4 | 5,4 | 4,8 | 4,4 | 4,1 | 3,8 | 3,5 | 3,2 | 3,0 | 2,8 | 2,6 | 2,4 |
| **300** | 24,7 | 24,2 | 24,0 | 23,8 | 23,6 | 23,4 | 23,2 | 23,1 | 23,0 | 22,9 | 22,8 | 22,6 |
| **200** | 35,0 | 35,0 | 34,8 | 34,7 | 34,6 | 34,5 | 34,5 | 34,4 | 34,4 | 34,4 | 34,4 | 34,3 |
| **100** | 33,3 | 34,6 | 35,4 | 36,1 | 36,6 | 36,9 | 37,4 | 37,6 | 37,8 | 38,1 | 38,3 | 38,5 |
| **<100** | 0,9 | 1,1 | 1,2 | 1,3 | 1,5 | 1,6 | 1,9 | 2,0 | 2,1 | 2,2 | 2,3 | 2,4 |

**Tabelle 1b: Siebanalyse der MgO 2-Kerne**

| **Sieb [µm]** | **Siebdauer [min]** | | | | | | | | | | | |
|---|---|---|---|---|---|---|---|---|---|---|---|---|
| | **5** | **10** | **15** | **20** | **25** | **30** | **35** | **40** | **45** | **50** | **55** | **60** |
| **800** | 0,1 | 0,1 | 0,1 | 0,1 | 0,0 | 0,1 | 0,1 | 0,1 | 0,1 | 0,1 | 0,0 | 0,1 |
| **600** | 14,6 | 12,3 | 11,0 | 9,8 | 9,0 | 8,3 | 7,7 | 7,2 | 6,6 | 6,1 | 5,7 | 5,3 |
| **500** | 18,1 | 17,5 | 16,7 | 15,8 | 15,2 | 14,5 | 13,8 | 13,2 | 12,6 | 11,9 | 11,4 | 10,7 |
| **400** | 15,7 | 15,4 | 14,9 | 14,3 | 13,8 | 13,3 | 12,8 | 12,2 | 11,7 | 11,1 | 10,6 | 10,1 |
| **300** | 20,9 | 21,0 | 20,9 | 20,6 | 20,3 | 20,1 | 19,8 | 19,4 | 19,1 | 18,6 | 18,2 | 17,8 |
| **200** | 21,6 | 22,9 | 23,7 | 24,6 | 25,1 | 25,6 | 26,1 | 26,6 | 27,0 | 27,4 | 27,8 | 28,2 |
| **100** | 5,6 | 6,8 | 7,9 | 8,9 | 9,7 | 10,4 | 11,2 | 11,9 | 12,6 | 13,3 | 14,0 | 14,7 |
| **<100** | 3,0 | 3,9 | 4,9 | 5,9 | 6,8 | 7,7 | 8,6 | 9,5 | 10,5 | 11,4 | 12,3 | 13,2 |

Alternativ hierzu, wurde die Abriebfestigkeit der MgO-Kerne auch noch mittels eines Einsaugversuches bestimmt bei dem 400 g der MgO-Kerne mittels Vakuums in ein Wirbelschichtgerät im Labor-Maßstab eingesaugt, und in diesem anschließend für ca. 5 min bei einer Temperatur von 25 °C und einer Luftmenge von 35 m³/h trocken verwirbelt wurden. In diesem Beispiel wurde hierfür ein Ventilus^{®}V-2.5 Gerät verwendet (Romaco Innojet, Steinen, Deutschland).

Zusätzlich hierzu wurde in beiden Versuchen (Siebanalyse und Einsaugversuch) die Partikelgröße der verwendeten Kerne mittels dynamischer Bildanalyse bestimmt, in einem Camsizer^{®} XT Gerät, ausgestattet mit der X-Jet Plug-in Kartusche sowie der dazugehörigen Analyse-Software (Retsch Technology GmbH, Haan, Deutschland). Die entsprechenden Daten - jeweils aus der Volumenverteilung bestimmt - sind in Tabelle 2 gelistet:

**Tabelle 2: Partikelgrößen zweier verschiedener Rohwaren von MgO Kernen**

| | **MgO 1** | | | **MgO 2** | | |
|---|---|---|---|---|---|---|
| | **a** | **b** | **c** | **a** | **b** | **c** |
| **D10** | 88,0 | 99,9 | 75,7 | 53,2 | 28,0 | 18,3 |
| **D50** | 223,4 | 225,4 | 222,3 | 354,7 | 264,5 | 294,4 |
| **Mittelwert** | 234,4 | 234,7 | 231,4 | 358,2 | 285,0 | 307,3 |
| **D90** | 399,4 | 390,2 | 397,6 | 644,3 | 588,3 | 630,4 |
| **Streuung** | 118,7 | 111,9 | 119,1 | 211,4 | 204,8 | 228,5 |
| **(D90-D10)/D50** | 1,39 | --- | --- | 1,67 | --- | --- |

a) ungestresst (Rohware der MgO-Kerne)
b) Abrieb nach 60 min Siebturm mit Siebhilfe (2-5 mm Silicagel; p = 750 kg/m³)
c) Abrieb nach 5 min Ventilus Einsaugversuch und Verwirbelung

Wie sich aus den Camsizer-Daten ablesen lässt, ist die MgO 2 Rohware der Magnesiumoxid-haltigen Kerne deutlich anfälliger für Abrieb und/oder Bruch der Kerne als die MgO 1 Rohware, und zeigt bei allen vier Parametern (D10, D90, Median D50 und Mittelwert der volumenbezogenen Partikelgrößenverteilung) eine Verkleinerung der Werte. Im Gegensatz hierzu bleiben die Werte der MgO 1-Rohware im Rahmen der Messstreuung überwiegend stabil, was auf eine eher geringe Neigung zu Staub-Abrieb oder Bruch der Kerne hinweist.

Dieselbe Tendenz ist auch aus den Abb. 1a und 1b ersichtlich: während der Feinanteil (<200 µm in Abb. 1a, und <300 µm in Abb. 1b) für die MgO 2 Kerne stetig und annähernd linear steigt und in Konsequenz die Masse an Kernen in den respektiven Partikelgrößen-Klassen 200-800 µm bzw. 300-800 µm stetig sinkt über den Verlauf der 60 min Siebdauer, ist für die MgO 1 Kerne nach einem initialen Abrieb während der ersten 15-20 min anschließend nur noch sehr moderater Abrieb zu verzeichnen. Jedoch selbst in diesen ersten 15-20 min ist der Abrieb für die MgO 1 Rohware deutlich geringer als für die MgO 2 Rohware.

In der Konsequenz ermöglicht die vorteilhafte erfindungsgemäße Abriebfestigkeit der MgO 1 Kerne ein Schmelzbeschichtungsverfahren, welches zuvor mit diversen anderen MgO Kernen nicht möglich war, bzw. nicht in reproduzierbarer Weise und nicht im industriellen Maßstab (bspw. ≥50 kg pro Charge) möglich war.

### Beispiel 2: Herstellung überzogener MgO-Retardpartikel im Labormaßstab

MgO 1 Kerne wie in Beispiel 1 (hier 0,8 kg) wurden in einem Wirbelschichtgerät (hier ein Ventilus V 2.5 ausgestattet mit einem Romaco Innojet Hot-Melt-Gerät IHD 1; Romaco Innojet, Steinen, Deutschland) im Hotmelt-Coating Verfahren mit geschmolzenem Glyceroltristearat (hier Dynasan^{®} 118) überzogen. Die Temperatur der Schmelze lag bei 90-100 °C, der Sprühdruck bei ca. 0,8-0,9 bar, die Sprührate ca. 6,5 g/min, und die Sprühluft-Temperatur bei ca. 100 °C.

Auf diese Weise wurden Überzugslevel von 30 Gew.-%, 20 Gew.-%, 15 Gew.-%, und 10 Gew.-% bezogen auf das Gewicht der überzogenen MgO-Retardpartikel hergestellt (d.h. 42,9 Gew.-%, 25,0 Gew.-%, 17,6 Gew.-%, und 11,1 Gew.-% bezogen auf das Ursprungsgewicht der nicht-überzogenen MgO-Kerne). Eine Menge von ca. 921 mg Retardpartikel mit einem Überzugslevel von 10 Gew.-% enthalten demnach ca. 829 mg MgO (entsprechend ca. 500 mg Mg) und ca. 92 mg des Dynasan^{®}118.

**Tabelle** 3 stellt die Partikelgrößen der Rohware (nicht-überzogene Kerne) im Vergleich mit der überzogenen Charge mit 10 % Überzugslevel dar, und deutet auf einen ungefähr 20 µm dicken Überzug hin. Zudem ist die Partikelgrößenverteilung der überzogenen Kerne etwas enger, wie der etwas geringere Quotient (D90-10)/D50 andeutet.

**Tabelle 3: Partikelgrößen von MgO 1 Kernen (nicht überzogen vs. Überzugslevel 10 %)**

| | **MgO 1** | **MgO 1 90:10** |
|---|---|---|
| **D10** | 95,5 | 136,8 |
| **D50** | 206,1 | 250,3 |
| **Mittelwert** | 217,0 | 259,5 |
| **D90** | 359,5 | 401,5 |
| **Streuung** | 101,2 | 105,8 |
| **(D90-D10)/D50** | 1,28 | 1,06 |

### Beispiel 3: Herstellung überzogener MgO-Retardpartikel im Industriemaßstab

MgO 1 Kerne wie in Beispiel 1 (hier 76,5 kg) wurden in einem Wirbelschichtgerät (hier ein Ventilus V 100 ausgestattet mit einem Romaco Innojet Hot-Melt-Gerät IHD 1; Romaco Innojet, Steinen, Deutschland) im Hotmelt-Coating Verfahren mit geschmolzenem Glyceroltristearat (hier Dnasan^{®} 118) überzogen. Die Temperatur der Schmelze lag bei 90-100 °C, der Sprühdruck bei ca. 1,0-1,2 bar, die Sprührate ca. 250 g/min, und die Sprühluft-Temperatur bei ca. 120 °C.

Auf diese Weise wurden Überzugslevel von 30 Gew.-%, 20 Gew.-%, 15 Gew.-%, und 10 Gew.-% bezogen auf das Gewicht der überzogenen MgO-Retardpartikel hergestellt (d.h. 42,9 Gew.-%, 25,0 Gew.-%, 17,6 Gew.-%, und 11,1 Gew.-% bezogen auf das Ursprungsgewicht der nicht-überzogenen MgO-Kerne).

### Beispiel 4: Herstellung oraler Direktgranulat-Formulierungen mit MgO Retardpartikeln

Die in Beispiel 2 erhaltenen MgO-Retardpartikel mit einem Überzugslevel von 15 Gew.-% oder 10 Gew.-% wurden mit verschiedenen Hilfsstoffmischungen versetzt (Tabelle 4 und Tabelle 5). Die Hilfsstoffmischungen wurden zunächst separat vermengt und anschließend den MgO-Retardpartikeln zugemischt. Der Gewichtsanteil der MgO-Retardpartikel am Gesamtgewicht der finalen Mischung, dem finalen oralen Direktgranulat, beträgt hierbei ca. 57 Gew.-% für die Mischung der Tabelle 4, und ca. 34 Gew.-% für die Mischung der Tabelle 5.

Die oralen Direktgranulate ODG 1-3 enthalten jeweils ca. 500 mg Magnesiumoxid in Retardpartikeln, 7,85 mg Trimagnesiumdicitrat in Form von nicht-retardierten Partikeln, sowie 10 µg Vit. D3 und 20 µg Vit. B12. Die drei Direktgranulate unterscheiden sich vorrangig in ihren Geschmacksgebern wie den Aromen, Zuckeralkoholen und/oder Süßstoffen.

Das orale Direktgranulat ODG 4 enthält ca. 350 mg Magnesiumoxid in Retardpartikeln, 42,15 mg Magnesiumhydrogencitrat und 7,85 mg Trimagnesiumdicitrat (beide in Form von nicht-retardierten Partikeln), sowie 1,1 mg Vit. B1, 1,4 mg Vit. B2 1,4 mg Vit. B6 und 2,5 µg Vit. B12.

**Tabelle 4: Zusammensetzung der zur Komponente (a) zugemischten Komponenten (Angaben in %)**

| **Bestandteile** | **ODG 1 MgO 1 85:15** | **ODG 2 MgO 1 85:15** | **ODG 3 MgO 1 90:10** |
|---|---|---|---|
| Sorbitol | 35,98 | 35,98 | 37,26 |
| Xylitol | 27,60 | 27,60 | 19,15 |
| Mononatriumcitrat (wasserfrei) | 7,59 | 7,59 | 14,73 |
| Calcium-Brausebasis | 6,90 | 6,90 | 7,37 |
| Trimagnesiumdicitrat (wasserfrei) | 6,90 | 6,90 | 7,37 |
| Zitronensäure | 4,83 | 4,83 | 2,95 |
| Cyanocobalamin (Vit. B12) 0,1% | 3,17 | 3,17 | 3,39 |
| Carmellose Natrium (Na-CMC) | 2,76 | 2,76 | 2,95 |
| Aroma Zitrone | 2,76 | --- | 2,95 |
| Aroma Multifrucht | --- | 2,76 | --- |
| Cholecalciferol (Vit. D3) | 0,75 | 0,75 | 0,80 |
| Magnesiumstearat | 0,55 | 0,55 | 0,59 |
| Aspartam | 0,21 | 0,21 | 0,44 |
| Acesulfam-K | --- | --- | 0,07 |

**Tabelle 5: Zusammensetzung der zur Komponente (a) zugemischten Komponenten (Angaben in %)**

| **Bestandteile** | **ODG 4 MgO 1 90:10** |
|---|---|
| Magnesiumhydrogencitrat | 39,51 |
| Sorbitol | 43,81 |
| Xylitol | 7,97 |
| Trimagnesiumdicitrat (wasserfrei) | 3,98 |
| Carmellose Natrium | 1,59 |
| Aroma Orange | 1,60 |
| Pyridoxin-HCl (Vit. B1) | 0,47 |
| Riboflavin (Vit. B2) | 0,38 |
| Thiaminnitrat (Vit. B6) | 0,37 |
| Cyanocobalamin (Vit. B12) 0,1% | 0,23 |
| Sucralose | 0,09 |

### Beispiel 5: Freisetzungsverhalten in 0.1 N Salzsäure

Das Freisetzungsverhalten von Magnesium aus den schmelzüberzogenen Retardpartikeln des Beispiels 2 mit einem Überzugslevel von 10 Gew.-% (90:10) bzw. 15 Gew.-% (85:15) sowie den damit formulierten erfindungsgemäßen oralen Direktgranulaten aus Beispiel 4 wurde in einer arzneibuchgemäßen Blattrührer-Freisetzungsapparatur (USP Dissolution Apparatus 2) bei 37 °C in 900 mL 0.1 N Salzsäure (HCl) bestimmt. Die Rührgeschwindigkeit betrug 75 UPM. Alle Versuche wurden mindestens sechs Mal wiederholt und Mittelwerte sowie Standard-abweichungen berechnet.

Wie aus Abb. 2 ersichtlich, ist die Freisetzung aus schmelzüberzogenen MgO 1-Kernen bei einem Überzugslevel von 15 Gew.-% ausreichend retardiert, um eine stabile Magnesium-versorgung über den gesamten Tag zu gewährleisten. Zudem sind die Freisetzungsprofile der beiden erfindungsgemäßen Zusammensetzungen ODG 1 und ODG 2 dem Freisetzungsprofil der bloßen schmelzüberzogenen MgO 1-Kernen sehr ähnlich, was darauf hinweist, dass die übrigen Bestandteile des Direktgranulates die Freisetzung aus den Retardpartikeln nicht negativ beeinflussen.

Wie aus Abb. 3 ersichtlich, ist die Freisetzung aus schmelzüberzogenen MgO 1-Kernen bei einem Überzugslevel von nur 10 Gew.-% wie erwartet etwas schneller als bei einem Überzugslevel von 15 Gew.-%; sie kann also mit dem Überzugslevel eingestellt werden. Beide sind jedoch ausreichend retardiert, um eine stabile Magnesiumversorgung über den gesamten Tag zu gewährleisten.

Abb. 4 zeigt die Freisetzung aus schmelzüberzogenen MgO 1-Kernen bei einem Überzugslevel von 10 Gew.-% für ein orales Direktgranulat welches ca. 50 mg nicht-retardiertes Magnesium und ca. 350 mg retardiertes Magnesium in Form der schmelzüberzogenen MgO 1-Kerne enthält (ODG 4), sowie im Vergleich hierzu ein orales Direktgranulat welches nur ca. 8 mg nicht-retardiertes Magnesium und ca. 500 mg retardiertes Magnesium in Form der schmelzüberzogenen MgO 1-Kerne enthält (ODG 3). Wie aus Abb. 4 ersichtlich, ist die Magnesium-Freisetzung des ODG 4 aufgrund des darin enthaltenen höheren nicht-retardierten Anteils schneller als für ODG 3 mit überwiegend retardierten Magnesium-Anteilen. Dies zeigt, dass die Magnesium-Freisetzung nicht nur mittels des Überzugslevels angepasst werden kann, sondern auch durch die Wahl des Anteils nicht-retardiertes versus retardiertes Magnesium.

## Patentansprüche

1. Feste pharmazeutische oder nutrazeutische Zusammensetzung in Form eines oralen Direktgranulats mit mindestens dualer Wirkstofffreisetzung, enthaltend folgende Komponenten:
(a) überzogene Retardpartikel mit einem Magnesium-haltigen Kern und einem Lipid-Überzug, wobei der Kern eine Magnesiumverbindung enthält oder aus dieser besteht, und wobei die Retardpartikel das enthaltene Magnesium in retardierter Weise freisetzen;
(b) nicht-retardierte Magnesium-haltige Partikel, wobei die Partikel eine Magnesiumverbindung enthalten oder aus dieser bestehen, und wobei die Partikel das enthaltene Magnesium in nicht-retardierter Weise freisetzen;
(c) einen oder mehrere wasserlösliche Hilfsstoffe ausgewählt aus der Gruppe der Zucker, Zuckeralkohole und Oligosaccharide; und
(d) optional einen oder mehrere weitere Hilfsstoffe;
**dadurch gekennzeichnet, dass** mindestens die Magnesium-haltigen Kerne der Retardpartikel der Komponente (a) eine solche Abriebfestigkeit aufweisen, dass nach einer Vibrationssiebung über einen Zeitraum von 60 Minuten:
― der Feinanteil an Partikeln mit einem Siebdurchmesser von < 200 µm nach einer Siebzeit von 60 Minuten, t₆₀ₘᵢₙ, um maximal 100 Gew.-%, bevorzugt um maximal 50 Gew.-%, weiter bevorzugt um maximal 20 Gew.-%, höher ist gegenüber dem initialen Feinanteil dieses Siebdurchmessers nach einer Siebzeit von 5 Minuten, t₅ₘᵢₙ; und/oder
― der Anteil an Partikeln mit einem Siebdurchmesser von 200 µm ≤ x < 800 µm nach einer Siebzeit von 60 Minuten, t₆₀ₘᵢₙ, um maximal 18 Gew.-%, bevorzugt um maximal 15 Gew.-%, weiter bevorzugt um maximal 12 Gew.-%, niedriger ist gegenüber dem initialen Anteil dieses Siebdurchmessers nach einer Siebzeit von 5 Minuten, t₅ₘᵢₙ.

2. Zusammensetzung nach Anspruch 1, wobei die Vibrationssiebung mit 100 g der Magnesium-haltigen Kerne und je 1 g Siebhilfe pro Siebboden sowie bei einer Vibrationsamplitude von 80/min durchgeführt wird.

3. Zusammensetzung nach einem der vorhergehenden Ansprüche, wobei die Magnesium-haltigen Kerne der Retardpartikel eine unimodale Partikelgrößenverteilung aufweisen; optional eine enge Partikelgrößenverteilung, bei welcher der Quotient (D90-D10) / D50 unter 1,60 liegt, bevorzugt unter 1,50, und weiter bevorzugt unter 1,40.

4. Zusammensetzung nach einem der vorhergehenden Ansprüche, wobei mindestens die Magnesiumverbindung in den Retardpartikeln der Komponente (a) in ihrer wasserfreien Form einen Magnesiumgehalt von 15 Gew.-% oder mehr aufweist, bevorzugt 25 Gew.-% oder mehr, weiter bevorzugt 50 Gew.-% oder mehr.

5. Zusammensetzung nach einem der vorhergehenden Ansprüche, wobei die Magnesiumverbindung in den Retardpartikeln ausgewählt ist aus Magnesiumoxid (MgO), Magnesiumcarbonat (MgCO₃), Magnesiumchlorid (MgCl₂), Magnesiumhydrogenphosphat (MgHPO₄) und/oder Magnesiumacetat (Mg(CH₃COO)₂).

6. Zusammensetzung nach einem der vorhergehenden Ansprüche, wobei der Überzug der Retardpartikel ein Lipid mit einem Schmelzpunkt von mindestens 50 °C enthält, oder im Wesentlichen hieraus besteht; bevorzugt ein Lipid mit einem Schmelzpunkt von mindestens 60 °C.

7. Zusammensetzung nach einem der vorhergehenden Ansprüche, wobei der Überzug der Retardpartikel mindestens 70 Gew.-%, oder mindestens 80 Gew.-%, oder mindestens 90 Gew.-% eines Triglycerids enthält.

8. Zusammensetzung nach einem der vorhergehenden Ansprüche, wobei der Überzug der Retardpartikel Glyceroltripalmitat und/oder Glyceroltristearat enthält, oder im Wesentlichen hieraus besteht.

9. Zusammensetzung nach einem der vorhergehenden Ansprüche, wobei das Gewichtsverhältnis zwischen Kern und Überzug in den Retardpartikeln zwischen 60:40 und 95:5 liegt, oder zwischen 70:30 und 90:10 liegt.

10. Zusammensetzung nach einem der vorhergehenden Ansprüche, wobei die Magnesiumverbindung in den nicht-retardierten Partikeln der Komponente (b) ausgewählt ist aus Magnesiumoxid (MgO), Magnesiumcarbonat (MgCO₃), Magnesiumacetat (Mg(CH₃COO)₂), Magnesiumhydrogenphosphat (MgHPO₄) Magnesiumhydrogencitrat, Trimagnesiumdicitrat (C₁₂H₁₀Mg₃O₁₄), Magnesiumpidolat (C₁₀H₁₂MgN₂O₆), Magnesium N-Acetyltaurinat (C₈H₁₆MgN₂O₈S₂) und/oder Magnesium-bis(hydrogen-L-glutamat), sowie deren Hydraten.

11. Zusammensetzung nach einem der vorhergehenden Ansprüche, wobei die Retardpartikel der Komponente (a) eine andere Magnesiumverbindung enthalten als die nicht-retardierten Partikel der Komponente (b).

12. Zusammensetzung nach einem der vorhergehenden Ansprüche, wobei die Retardpartikel der Komponente (a), und optional die nicht-retardierten Partikel der Komponente (b), eine mediane Teilchengröße (D50), gemessen mittels dynamischer Bildanalyse, von ≤ 500 µm aufweisen, bevorzugt ≤ 450 µm, weiter bevorzugt ≤ 400 µm.

13. Zusammensetzung nach einem der vorhergehenden Ansprüche, wobei die Retardpartikel der Komponente (a) zwischen 15 und 85 Gew.-%, oder zwischen 25 und 75 Gew.-%, oder zwischen 30 und 70 Gew.-%, oder zwischen 30 und 60 Gew.-% der Zusammensetzung ausmachen.

14. Verfahren zur Herstellung einer festen pharmazeutischen oder nutrazeutischen Zusammensetzung nach den Ansprüchen 1 bis 13, enthaltend die folgenden Schritte:
(i) Bereitstellen eines Magnesium-haltigen Kerns, wobei der Kern eine Magnesiumverbindung enthält oder aus dieser besteht;
(ii) Bereitstellen eines geschmolzenen Überzugsmaterials enthaltend ein Lipid;
(iii) Fluidisieren der Magnesium-haltigen Kerne;
(iv) Besprühen der fluidisierten Magnesium-haltigen Kerne mit dem geschmolzenen Überzugsmaterial;
(v) Abkühlen der überzogenen Magnesium-haltigen Kerne, so dass das Lipid erstarrt und Retardpartikel gemäß Komponente (a) erhalten werden, welche das enthaltene Magnesium in retardierter Weise freisetzen; und
(vi) Mischen der so als Komponente (a) erhaltenen Retardpartikel mit den folgenden weiteren Komponenten:
(b) nicht-retardierte Partikel mit einem zweiten Magnesium-haltigen Kern, wobei der Partikel eine Magnesiumverbindung enthält oder aus dieser besteht, und wobei die Partikel das enthaltene Magnesium in nicht-retardierter Weise freisetzen;
(c) einen oder mehrere wasserlösliche Hilfsstoffe ausgewählt aus der Gruppe der Zucker, Zuckeralkohole und Oligosaccharide; und
(d) optional weiteren Hilfsstoffen;
**dadurch gekennzeichnet, dass** mindestens die Magnesium-haltigen Kerne der Retardpartikel der Komponente (a) eine solche Abriebfestigkeit aufweisen, dass nach einer Vibrationssiebung über einen Zeitraum von 60 Minuten:
― der Feinanteil an Partikeln mit einem Siebdurchmesser von < 200 µm nach einer Siebzeit von 60 Minuten, t₆₀ₘᵢₙ, um maximal 100 Gew.-%, bevorzugt um maximal 50 Gew.-%, weiter bevorzugt um maximal 20 Gew.-%, höher ist gegenüber dem initialen Feinanteil dieses Siebdurchmessers nach einer Siebzeit von 5 Minuten, t₅ₘᵢₙ; und/oder
― der Anteil an Partikeln mit einem Siebdurchmesser von 200 µm ≤ x < 800 µm nach einer Siebzeit von 60 Minuten, t₆₀ₘᵢₙ, um maximal 18 Gew.-%, bevorzugt um maximal 15 Gew.-%, weiter bevorzugt um maximal 12 Gew.-%, niedriger ist gegenüber dem initialen Anteil dieses Siebdurchmessers nach einer Siebzeit von 5 Minuten, t₅ₘᵢₙ.

15. Feste pharmazeutische oder nutrazeutische Zusammensetzung in Form eines oralen Direktgranulats mit mindestens dualer Wirkstofffreisetzung, hergestellt durch das Verfahren nach Anspruch 14.
